# EUROPEAN PATENT APPLICATION

(11) **EP 4 435 006 A1**
(43) Date of publication of application: **25.09.2024**
(21) Application number: 21964409.3
(22) Date of filing: 19.11.2021
(51) Int. Cl.: C07K 16/28, C07K 19/00, C12N 15/13, A61K 39/395, A61P 35/00

(54) **BISPECIFIC ANTIBODY AND USE THEREOF**

(71) Applicant: Wuhan Yzy Biopharma Co., Ltd., Wuhan, Hubei 430075 (CN)
(72) Inventor: FANG, Lijuan, Wuhan, Hubei 430075 (CN); ZHANG, Jing, Wuhan, Hubei 430075 (CN); HUA, Shan, Wuhan, Hubei 430075 (CN); ZHOU, Pengfei, Wuhan, Hubei 430075 (CN)
(74) Representative: Altmann Stößel Dick Patentanwälte PartG mbB
(86) International application number: PCT/CN2021/131804
(87) International publication number: WO 2023/087255

(57) **Abstract**

Provided in the present invention are a bispecific antibody and the use thereof. The bispecific antibody comprises an antigen-binding domain specifically binding to EpCAM and an antigen-binding domain specifically binding to CD3.

## Description

### TECHNICAL FIELD

The present invention relates to the technical field of immunology, and in particular, relates to a bispecific antibody against EpCAM and CD3, and use thereof.

### BACKGROUND

Bispecific antibodies (BsAbs), also known as dual-targeting antibodies, can simultaneously recognize and bind to two different antigens or epitopes and block two different signaling pathways to exert their effects. Compared to monoclonal antibodies (monoclonal Abs, mAbs) that recognize a single antigen, bispecific antibodies have numerous advantages: (1) the ability to redirect specific immune effector cells to neighboring tumor cells to enhance tumor killing, which is not achievable by mAb combination treatment strategies; (2) increased binding specificity through the interaction of two different cell surface antigens; (3) the ability to reduce the costs of development and the budgets for clinical trials and regulatory reviews compared to the drug development of monoclonal antibodies in combination therapies; (4) the ability to simultaneously block two different pathways that exert unique or overlapping functions in pathogenesis compared to drugs of monoclonal antibodies in combination therapies.

Cancer and other diseases are all multifactorial and have many signaling pathways in etiology, so that single-target immunotherapy cannot effectively kill targeted cells. Patients receiving mAb treatment may develop drug resistance or may not respond to the treatment. Accordingly, bispecific antibodies have become a major option for the treatment of many diseases such as cancer, inflammation, viral infections, and autoimmune diseases. However, bispecific antibodies do not exist in the natural environment and need to be realized by recombinant DNA, cell fusion or chemical binding technologies, among which the recombinant DNA technology is the most commonly used technology for preparing BsAbs at present, but there are still many obstacles such as difficulty in expression, low yield, difficulty in purification and poor stability of BsAbs. Therefore, it is very necessary to construct a novel bispecific antibody that can overcome the above obstacles and to establish a corresponding animal model for immune killing. The present invention provides a novel bispecific antibody and describes the methods and results for its pharmacodynamics studies.

### SUMMARY

The present invention has developed a novel bispecific antibody, comprising an antigen-binding domain specifically binding to EpCAM and an antigen-binding domain specifically binding to CD3, and use thereof.

Specifically, the present invention relates to the following aspects:
1. A bispecific antibody, comprising an antigen-binding domain specifically binding to EpCAM and an antigen-binding domain specifically binding to CD3,
   wherein the antigen-binding domain specifically binding to EpCAM is selected from the group consisting of:
      1) an antigen-binding domain specifically binding to EpCAM, which comprises the following CDRs or variants thereof:
         (i) CDRH1, CDRH2 and CDRH3 comprised in a heavy chain variable region set forth in SEQ ID NO: 14, and
         (ii) CDRL1, CDRL2 and CDRL3 comprised in a light chain variable region set forth in SEQ ID NO: 13,
         wherein preferably, according to the Kabat sequence numbering system, the sequence of CDRL1 is set forth in SEQ ID NO: 32, the sequence of CDRL2 is set forth in SEQ ID NO: 33, the sequence of CDRL3 is set forth in SEQ ID NO: 34, the sequence of CDRH1 is set forth in SEQ ID NO: 35, the sequence of CDRH2 is set forth in SEQ ID NO: 36, and the sequence of CDRH3 is set forth in SEQ ID NO: 37; or
      2) an antigen-binding domain specifically binding to EpCAM, which comprises the following CDRs or variants thereof:
         (i) CDRH1, CDRH2 and CDRH3 comprised in a heavy chain variable region set forth in SEQ ID NO: 16, and
         (ii) CDRL1, CDRL2 and CDRL3 comprised in a light chain variable region set forth in SEQ ID NO: 15,
   wherein preferably, according to the Kabat sequence numbering system and CDR definition system, the sequence of CDRL1 is set forth in SEQ ID NO: 38, the sequence of CDRL2 is set forth in SEQ ID NO: 39, the sequence of CDRL3 is set forth in SEQ ID NO: 40, the sequence of CDRH1 is set forth in SEQ ID NO: 41, the sequence of CDRH2 is set forth in SEQ ID NO: 42, and the sequence of CDRH3 is set forth in SEQ ID NO: 43;
   the antigen-binding domain specifically binding to CD3 is selected from the group consisting of:
      1) an antigen-binding domain specifically binding to CD3, which comprises the following CDRs or variants thereof:
         CDRH1, CDRH2 and CDRH3 comprised in a heavy chain variable region set forth in SEQ ID NO: 50, and CDRL1, CDRL2 and CDRL3 comprised in a light chain variable region set forth in SEQ ID NO: 51,
         wherein preferably, according to the Kabat sequence numbering system, the sequence of CDRH1 is set forth in SEQ ID NO: 44, the sequence of CDRH2 is set forth in SEQ ID NO: 45, the sequence of CDRH3 is set forth in SEQ ID NO: 46, the sequence of CDRL1 is set forth in SEQ ID NO: 47, the sequence of CDRL2 is set forth in SEQ ID NO: 48, and the sequence of CDRL3 is set forth in SEQ ID NO: 49; or
      2) an antigen-binding domain specifically binding to CD3, which comprises the following CDRs or variants thereof:
         CDRH1, CDRH2 and CDRH3 comprised in a heavy chain variable region set forth in SEQ ID NO: 58, and CDRL1, CDRL2 and CDRL3 comprised in a light chain variable region set forth in SEQ ID NO: 59,
         wherein preferably, according to the Kabat sequence numbering system, the sequence of CDRH1 is set forth in SEQ ID NO: 52, the sequence of CDRH2 is set forth in SEQ ID NO: 53, the sequence of CDRH3 is set forth in SEQ ID NO: 54, the sequence of CDRL1 is set forth in SEQ ID NO: 55, the sequence of CDRL2 is set forth in SEQ ID NO: 56, and the sequence of CDRL3 is set forth in SEQ ID NO: 57; the variants of the CDRs differ from the corresponding CDRs by 3, 2 or 1 amino acid or have at least 80%, 85%, 90%, 91%, 92%, 93%, 94%, 95%, 96%, 97%, 98% or 99% identity to the corresponding CDRs.
2. The bispecific antibody according to item 1, wherein the antigen-binding domain specifically binding to EpCAM comprises the following heavy chain variable region and light chain variable region (or variants thereof):
   (i) a heavy chain variable region set forth in SEQ ID NO: 14 and a light chain variable region set forth in SEQ ID NO: 13; or
   (ii) a heavy chain variable region set forth in SEQ ID NO: 16 and a light chain variable region set forth in SEQ ID NO: 15; and

   the antigen-binding domain specifically binding to CD3 comprises the following heavy chain variable region and light chain variable region (or variants thereof):
      (1) a heavy chain variable region set forth in SEQ ID NO: 50 and a light chain variable region set forth in SEQ ID NO: 51, or
      (2) a heavy chain variable region set forth in SEQ ID NO: 58 and a light chain variable region set forth in SEQ ID NO: 59;
   preferably, the antigen-binding domain specifically binding to EpCAM is in the form of a Fab fragment and the antigen-binding domain specifically binding to CD3 is in the form of an ScFv;
   preferably, the antigen-binding domain specifically binding to EpCAM comprises the following heavy chain variable region and light chain variable region (or variants thereof):
      (i) a heavy chain variable region set forth in SEQ ID NO: 14 and a light chain variable region set forth in SEQ ID NO: 13; and
   the antigen-binding domain specifically binding to CD3 is selected from the group consisting of:
      (1) an ScFv set forth in SEQ ID NO: 18 or a variant thereof,
      (2) an ScFv set forth in SEQ ID NO: 19 or a variant thereof,
   wherein the variants differ from the corresponding variable regions or ScFvs by 3, 2 or 1 amino acid or have at least 80%, 85%, 90%, 91%, 92%, 93%, 94%, 95%, 96%, 97%, 98% or 99% identity to the corresponding variable regions or ScFvs.
3. The bispecific antibody according to item 1 or 2, wherein the bispecific antibody comprises:
   (1) a light chain-heavy chain pair specifically binding to EpCAM, the light chain-heavy chain pair comprising or consisting of a light chain and a heavy chain, wherein the light chain comprises a light chain variable region and a light chain constant region (a preferred sequence is set forth in any one of SEQ ID NOs: 1 and 60-65), and the heavy chain comprises a heavy chain variable region, CH1 (a preferred sequence is set forth in SEQ ID NO: 2) and a first Fc fragment, wherein preferably, the first Fc fragment comprises a hinge region (a preferred sequence is set forth in SEQ ID NO: 3), CH2 (a preferred sequence is set forth in any one of SEQ ID NOs: 6, 7 and 66-71) and CH3a; and
   (2) a fusion peptide specifically binding to CD3, the fusion peptide comprising or consisting of an ScFv specifically binding to CD3 and a second Fc fragment, wherein preferably, the ScFv comprises, in sequence from the N-terminus to the C-terminus, a heavy chain variable region, a linker peptide (a preferred sequence is set forth in SEQ ID NO: 4) and a light chain variable region, and the second Fc fragment comprises, in sequence from the N-terminus to the C-terminus, a hinge region (a preferred sequence is set forth in SEQ ID NO: 3), CH2 (a preferred sequence is set forth in any one of SEQ ID NOs: 6, 7 and 66-71) and CH3b, wherein preferably, the C-terminus of the light chain variable region is linked to the hinge region of the second Fc fragment via a linker peptide (a preferred sequence is set forth in SEQ ID NO: 5);

   preferably, the first Fc fragment and the second Fc fragment are human or humanized Fc fragments, such as human IgG Fc fragments, e.g., IgG1, IgG2, IgG3, IgG4 or IgG5 Fc fragments;
   preferably, compared to a wild-type antibody, the first Fc fragment and/or the second Fc fragment comprise one or more substitutions, that form a knob-into-hole structure pair between the heavy chain and the fusion peptide, e.g., T366 on one CH3 domain is substituted with a relatively larger amino acid residue, such as tyrosine (Y) or tryptophan (W), while Y407 on the other CH3 domain is substituted with a relatively smaller amino acid residue, such as threonine (T), alanine (A) or valine (V); for example, the first Fc fragment and/or the second Fc fragment comprise one or more substitutions in Table 6;
   preferably, the first Fc fragment and/or the second Fc fragment comprise one or more substitutions, wherein 1) the substitution forms a salt bridge pair between the heavy chain and the fusion peptide, e.g., one CH3 domain comprises one or more substitutions with an amino acid residue that is positively charged under physiological conditions, while the other CH3 domain comprises one or more substitutions with one or more amino acid residues that are negatively charged under physiological conditions, wherein the positively charged amino acid residue is, for example, arginine (R), histidine (H) or lysine (K), the negatively charged amino acid residue may be, for example, aspartic acid (D) or glutamic acid (E), and the substituted amino acid residues comprise, for example, one or more of D356, L368, K392, D399 and K409, for example, one or more substitutions in Table 7; 2) the substitution forms a disulfide bond between the heavy chain and the fusion peptide, for example the substitutions in Table 8; and/or 3) the substitution results in a significant reduction in binding ability between the Fc and protein A, for example, H435 and Y436 on one CH3 domain are substituted with arginine and phenylalanine, respectively, as shown in Table 9; wherein preferably:
      a) the CH3b of the fusion peptide and the CH3a of the heavy chain have a substitution pair that forms a knob-into-hole structure;
      b) the CH3b of the fusion peptide and the CH3a of the heavy chain have a substitution pair that forms an ionic bond;
      c) the CH3b of the fusion peptide and the CH3a of the heavy chain have a substitution pair that forms a disulfide bond; and/or
      d) the CH3b of the fusion peptide and the CH3a of the heavy chain have a substitution that results in reduced binding ability to the protein A;
   preferably, the CH1 comprises a sequence of SEQ ID NO: 2; and/or CL comprises a sequence selected from any one of SEQ ID NOs: 1 and 60-65;
   preferably, the first Fc fragment and/or the second Fc fragment comprise CH2 of a sequence selected from any one of SEQ ID NOs: 6, 7 and 66-71, and/or CH3 of a sequence selected from any one of SEQ ID NOs: 8, 9, 11, 12 and 72-76;
   preferably, the sequences of CH3a and the CH3b are selected from the group consisting of:
      (1) sequences in which one is set forth in SEQ ID NO: 8 and the other is set forth in SEQ ID NO: 11;
      (2) sequences in which one is set forth in SEQ ID NO: 9 and the other is set forth in SEQ ID NO: 12;
      (3) sequences in which one is set forth in SEQ ID NO: 72 and the other is set forth in SEQ ID NO: 74;
      (4) sequences in which one is set forth in SEQ ID NO: 9 and the other is set forth in SEQ ID NO: 75;
      (5) sequences in which one is set forth in SEQ ID NO: 73 and the other is set forth in SEQ ID NO: 76;
   preferably, the bispecific antibody is selected from the group consisting of:
      (1) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 11, the heavy chain comprises or consists of SEQ ID NO: 14, SEQ ID NO: 2, SEQ ID NO: 3, SEQ ID NO: 6 and SEQ ID NO: 8, and the light chain comprises or consists of SEQ ID NO: 13 and SEQ ID NO: 1;
      (2) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 11, the heavy chain comprises or consists of SEQ ID NO: 14, SEQ ID NO: 2, SEQ ID NO: 3, SEQ ID NO: 6 and SEQ ID NO: 8, and the light chain comprises or consists of SEQ ID NO: 13 and SEQ ID NO: 1;
      (3) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 11, the heavy chain comprises or consists of SEQ ID NO: 16, SEQ ID NO: 2, SEQ ID NO: 3, SEQ ID NO: 6 and SEQ ID NO: 8, and the light chain comprises or consists of SEQ ID NO: 15 and SEQ ID NO: 1;
      (4) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 11, the heavy chain comprises or consists of SEQ ID NO: 16, SEQ ID NO: 2, SEQ ID NO: 3, SEQ ID NO: 6 and SEQ ID NO: 8, and the light chain comprises or consists of SEQ ID NO: 15 and SEQ ID NO: 1;
      (5) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 7 and SEQ ID NO: 12, the heavy chain comprises or consists of SEQ ID NO: 14, SEQ ID NO: 2, SEQ ID NO: 3, SEQ ID NO: 7 and SEQ ID NO: 9, and the light chain comprises or consists of SEQ ID NO: 13 and SEQ ID NO: 1;
      (6) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 7 and SEQ ID NO: 12, the heavy chain comprises or consists of SEQ ID NO: 14, SEQ ID NO: 2, SEQ ID NO: 3, SEQ ID NO: 7 and SEQ ID NO: 9, and the light chain comprises or consists of SEQ ID NO: 13 and SEQ ID NO: 1;
      (7) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 8, the heavy chain comprises or consists of SEQ ID NO: 14, SEQ ID NO: 2, SEQ ID NO: 3, SEQ ID NO: 6 and SEQ ID NO: 11, and the light chain comprises or consists of SEQ ID NO: 13 and SEQ ID NO: 1;
      (8) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 8, the heavy chain comprises or consists of SEQ ID NO: 14, SEQ ID NO: 2, SEQ ID NO: 3, SEQ ID NO: 6 and SEQ ID NO: 11, and the light chain comprises or consists of SEQ ID NO: 13 and SEQ ID NO: 1;
      (9) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 8, the heavy chain comprises or consists of SEQ ID NO: 16, SEQ ID NO: 2, SEQ ID NO: 3, SEQ ID NO: 6 and SEQ ID NO: 11, and the light chain comprises or consists of SEQ ID NO: 15 and SEQ ID NO: 1;
      (10) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 8, the heavy chain comprises or consists of SEQ ID NO: 16, SEQ ID NO: 2, SEQ ID NO: 3, SEQ ID NO: 6 and SEQ ID NO: 11, and the light chain comprises or consists of SEQ ID NO: 15 and SEQ ID NO: 1;
      (11) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 7 and SEQ ID NO: 9, the heavy chain comprises or consists of SEQ ID NO: 14, SEQ ID NO: 2, SEQ ID NO: 3, SEQ ID NO: 7 and SEQ ID NO: 12, and the light chain comprises or consists of SEQ ID NO: 13 and SEQ ID NO: 1; and
      (12) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 7 and SEQ ID NO: 9, the heavy chain comprises or consists of SEQ ID NO: 14, SEQ ID NO: 2, SEQ ID NO: 3, SEQ ID NO: 7 and SEQ ID NO: 12, and the light chain comprises or consists of SEQ ID NO: 13 and SEQ ID NO: 1.
4. A nucleic acid composition, comprising a nucleic acid sequence encoding the bispecific antibody according to any one of items 1-3, wherein preferably,
   the nucleic acid composition comprises:
   a) a first expression vector comprising a first nucleic acid encoding the antigen-binding domain or light chain-heavy chain pair specifically binding to EpCAM as defined in any one of items 1-3;
   b) a second expression vector comprising a second nucleic acid encoding the antigen-binding domain or fusion peptide specifically binding to CD3 as defined in any one of items 1-3.
5. An expression vector, comprising the nucleic acid composition according to item 4.
6. A host cell, comprising the expression vector according to item 5.
7. A pharmaceutical composition, comprising the bispecific antibody according to any one of items 1-3, and a pharmaceutically acceptable carrier, and optionally, a drug (e.g., a small molecule drug or a large molecule drug) for treating cancer (an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, oral cancer, or the like) and/or malignant ascites, malignant effusion, malignant pleural effusion, or the like, wherein preferably, the dosage form of the pharmaceutical composition comprises a dosage form for gastrointestinal administration or a dosage form for parenteral administration; and more preferably, the dosage form of the pharmaceutical composition is an injectable form, including intravenous injection, intravenous drip, subcutaneous injection, topical injection, intramuscular injection, intratumoral injection, intraperitoneal injection, intracranial injection, or intracavitary injection.
8. A conjugate or fusion protein, comprising the bispecific antibody according to any one of items 1-3, preferably comprising a substance A conjugated or fused to the bispecific antibody, wherein the substance A is selected from a therapeutic agent, a prodrug, a protein (e.g., an enzyme), a virus, a lipid, a biological response modifier (e.g., an immunomodulator), PEG, a hormone, an oligonucleotide, a diagnostic agent, a cytotoxic agent that can be a drug or a toxin, an ultrasound enhancing agent, a non-radioactive label, a detectable label such as a chemiluminescent labeling compound (e.g., luminol, isoluminol, theromatic acridinium ester, imidazole, acridinium salt, and oxalate), or a fluorescent luminescent metal (e.g., 152Eu, or a lanthanide label).
9. A kit, comprising the bispecific antibody according to any one of items 1-3 and optionally, a drug (e.g., a small molecule drug or a large molecule drug) for treating cancer (an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, oral cancer, or the like) and/or malignant ascites, malignant effusion, malignant pleural effusion, or the like.
10. Use of the bispecific antibody according to any one of items 1-3 in the treatment of cancer or in the preparation of a medicament or kit for treating cancer and/or malignant ascites, malignant effusion, malignant pleural effusion, or the like, wherein the cancer is, for example, an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, or oral cancer.
11. A method for treating cancer and/or malignant ascites, malignant effusion, or malignant pleural effusion, comprising administering to a subject a therapeutically effective amount of the bispecific antibody according to any one of items 1-3, wherein the cancer is an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, or oral cancer.

It should be understood that within the scope of the present invention, the above technical features of the present invention and the technical features specifically described hereinafter (as in the examples) may be combined with each other to constitute a new or preferred technical solution. Due to the limited space, such solutions are not described herein.

The terms referred to in the present invention have the conventional meanings understood by those skilled in the art. When a term has two or more definitions as used and/or acceptable in the art, the definition of the term used herein is intended to include all meanings.

It will be understood by those of ordinary skills in the art that the CDRs of an antibody are responsible for the binding specificity of the antibody to an antigen. For a given heavy or light chain variable region sequence of an antibody, there are several methods for determining the CDRs of the antibody, including the Kabat, IMGT, Chothia and AbM numbering systems. However, the application of all the definitions of CDRs for an antibody or variants thereof shall fall within the scope of the terms defined and used herein. If the amino acid sequence of the variable region of the antibody is given, those skilled in the art can generally determine a particular CDR, without relying on any experimental data beyond the sequence itself.

As used herein, "antibody" or "antigen-binding fragment" refers to a polypeptide or polypeptide complex that specifically recognizes and binds to an antigen. The term "antibody" is used in a broad sense and includes immunoglobulins or antibody molecules including monoclonal or polyclonal human, humanized, complex and chimeric antibodies, and antibody fragments. Thus, the term "antibody" includes any protein or peptide comprising a specific molecule that comprises at least a portion of an immunoglobulin molecule having biological activity for binding to an antigen. Such examples include, but are not limited to, complementarity determining regions (CDRs) of a heavy or light chain or a ligand binding portion thereof, heavy or light chain variable regions, heavy or light chain constant regions, framework regions (FRs) or any portion thereof, or at least a portion of a binding protein. In the present invention, the antibody includes murine, chimeric, humanized or fully human antibodies prepared by using techniques well known to those skilled in the art. Recombinant antibodies, such as chimeric and humanized monoclonal antibodies, including human and non-human portions, can be prepared by using recombinant DNA technology well known to those skilled in the art. The immunoglobulin molecules or antibody molecules of the present application may be of any type (e.g., IgG, IgE, IgM, IgD, IgA, and IgY), or any class (e.g., IgG1, IgG2, IgG3, IgG4, IgA1, and IgA2) or subclass of immunoglobulin molecules.

The term "antibody fragment" or "antigen-binding fragment" includes, but is not limited to, F(ab')₂, F(ab)₂, Fab', Fab, Fv, Fd, dAb, Fab/c, complementarity determining region (CDR) fragment, single-chain Fv (ScFv), disulfide-stabilized Fv fragment (dsFv), (dsFv)₂, bispecific dsFv (dsFv-dsFv'), diabody, disulfide-stabilized diabody (ds-diabody), ScFv multimer (e.g., ScFv dimer and ScFv trimer), multispecific antibody formed from a portion of an antibody comprising one or more CDRs, nanobody, single-domain antibody (sdab), domain antibody, bivalent domain antibody, or any other antibody fragment that binds to an antigen but does not comprise an intact antibody structure. Regardless of the structure, the antigen-binding fragment includes any polypeptide or polypeptide complex capable of binding to the same antigen to which the parent antibody or the parent antibody fragment binds. The term "antibody fragment" includes aptamers, spiegelmers, and diabodies. The term "antibody fragment" also includes any synthetic or genetically engineered protein that, like antibodies, binds to a specific antigen to form a complex. Typically, the antibody fragment has at least about 50 contiguous amino acids, preferably at least about 50 contiguous amino acids, more preferably at least about 80 contiguous amino acids, and most preferably at least about 100 contiguous amino acids, of the antibody of the present invention.

"Single-chain variable fragment" or "ScFv" refers to a fusion protein of the variable regions of the heavy chain (VH) and light chain (VL) of an immunoglobulin. In certain aspects, these regions are linked via a short linker peptide of 10 to about 25 amino acids. The linker may be rich in glycine to provide flexibility, may also contain serine or threonine to provide solubility, and is capable of linking the N-terminus of the VH to the C-terminus of the VL, and vice versa. This protein retains the properties of the original immunoglobulin except for the removal of the constant region and the introduction of the linker. ScFv molecules are known in the art, such as those described in the U.S. Patent No. 5,892,019.

An antigen-binding domain binding to EpCAM and CD3 is a Fab, or an ScFv, or a non-covalent pair (Fv) between a heavy chain variable region (VH) and a light chain variable region (VL). Any of the above antibodies or polypeptides may also comprise additional polypeptides, e.g., a signal peptide at the N-terminus of the antibody, which is used to direct secretion, or other heterologous polypeptides as described herein, such as a 6×His tag used for purification. In addition to intact antibodies, the present invention further includes immunocompetent antibody fragments or fusion proteins formed by the antibodies and other sequences. The present invention further provides other proteins or fusion expression products having the antibody of the present invention. Specifically, the present invention includes any protein or protein conjugate and fusion expression product (i.e., immunoconjugate and fusion expression product) having heavy and light chains with variable regions, so long as the variable regions are identical or have at least 90% homology, preferably at least 95% homology, most preferably 96%, 97%, 98%, 99% or more homology, to the variable regions of the heavy and light chains of the antibody of the present invention. Thus, the present invention includes molecules having monoclonal antibody light and heavy chain variable regions with CDRs, so long as the CDRs have 90% or more (preferably 95% or more, most preferably 96%, 97%, 98%, 99% or more) homology to the CDRs of the present invention.

The present invention further includes fragments, variants, derivatives and analogs of the antibodies. The antibody, the antigen-binding fragment, or the variants or derivatives thereof of the present application include, but are not limited to, polyclonal antibody, monoclonal antibody, multispecific antibody (e.g., bispecific antibody, trispecific antibody, and the like), human antibody, antibody of animal source, humanized antibody, primatized antibody, or chimeric antibody, CDR-grafted and/or modified antibody, single-chain antibody (e.g., ScFv), diabody, epitope-binding fragments such as Fab, Fab' and F(ab')₂, Fd, Fv, single-chain Fv (ScFv), single-chain antibody, disulfide-stabilized Fv (dsFv), fragments comprising VL or VH domains, fragments produced from Fab expression libraries, and anti-idiotypic (anti-Id) antibody. The antibody fragment, the antigen-binding fragment, the derivative or the analog of the present invention may be (i) a polypeptide in which one or more conservative or non-conservative amino acid residues (preferably conservative amino acid residues) are substituted, and such substituted amino acid residues may or may not be encoded by the genetic codes, or (ii) a polypeptide having a substituted group in one or more amino acid residues, or (iii) a polypeptide formed by fusing a mature polypeptide with another compound (such as a compound that prolongs the half-life of the polypeptide, e.g., polyethylene glycol), or (iv) a polypeptide formed by fusing an additional amino acid sequence with the polypeptide sequence (such as a leader sequence, a secretion sequence, a sequence for purifying the polypeptide, a proteinogenic sequence, or a fusion protein formed with a 6×His tag). Such fragments, derivatives and analogs are well known to those skilled in the art according to the teachings of the present invention.

The antibody of the present invention refers to a polypeptide having binding activity to human EpCAM and CD3, and comprising the above CDRs. The term further includes variants of the polypeptides having the same function as the antibody of the present invention and comprising the above CDRs. These variations include (but are not limited to): deletion, insertion and/or substitution of one or more (generally 1-50, preferably 1-30, more preferably 1-20, and most preferably 1-10) amino acids, and addition of one or more (generally within 20, preferably within 10, and more preferably within 5) amino acids to the C-terminus and/or N-terminus. For example, in the art, substitutions with amino acids that are similar in properties generally do not alter the function of the protein. For another example, the addition of one or more amino acids to the C-terminus and/or N-terminus generally does not alter the function of the protein. The term further includes active fragments and active derivatives of the antibody of the present invention. Variants of the polypeptide include: homologous sequences, conservative variants, allelic variants, natural mutants, induced mutants, proteins encoded by DNA capable of hybridizing to DNA encoding the antibody of the present invention under high- or low-stringency conditions, and polypeptides or proteins obtained by using antisera against the antibody of the present invention.

The antibody of the present invention may be (i) a polypeptide in which one or more conservative or non-conservative amino acid residues (preferably conservative amino acid residues) are substituted, or (ii) a polypeptide having a substituted group in one or more amino acid residues, or (iii) a polypeptide formed by fusing a mature polypeptide with another compound (such as a compound that prolongs the half-life of the polypeptide, e.g., polyethylene glycol), or (iv) a polypeptide formed by fusing an additional amino acid sequence with the polypeptide sequence (such as a leader sequence, a secretion sequence, a sequence for purifying the polypeptide, a proteinogenic sequence, or a fusion protein formed with a 6His tag). Such fragments, derivatives and analogs are well known to those skilled in the art according to the teachings of the present invention.

"Conservative amino acid substitution" is one in which an amino acid residue is substituted with an amino acid residue having a similar side chain. Families of amino acid residues having similar side chains have been defined in the art, including basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), non-polar side chains (e.g., alanine, valine, leucine, isoleucine, proline, phenylalanine, methionine, and tryptophan), branched β side chains (e.g., threonine, valine and isoleucine), and aromatic side chains (e.g., tyrosine, phenylalanine, tryptophan, and histidine). Thus, non-essential amino acid residues of an immunoglobulin polypeptide are preferably substituted with other amino acid residues from the same side chain family. In other embodiments, a string of amino acids may be substituted with a structurally similar string of amino acids that differ in sequence and/or composition of the side chain family.

Non-limiting examples of conservative amino acid substitutions are provided in the following table, where a similarity score of 0 or higher indicates that there is a conservative substitution between the two amino acids.

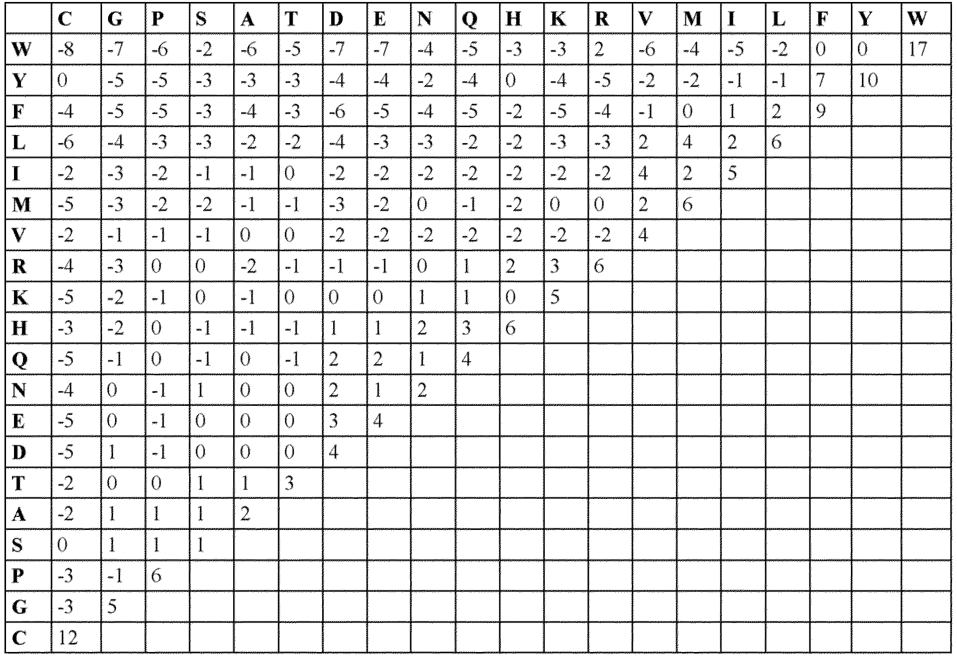

In some embodiments, the conservative substitution is preferably a substitution in which one amino acid within the following groups (a)-(e) is substituted with another amino acid residue within the same group: (a) small aliphatic, non-polar or weakly polar residues: Ala, Ser, Thr, Pro and Gly, (b) polar, negatively charged residues and (uncharged) amides thereof: Asp, Asn, Glu and Gin, (c) polar, positively charged residues: His, Arg and Lys, (d) bulky aliphatic, nonpolar residues: Met, Leu, Ile, Val and Cys, and (e) aromatic residues: Phe, Tyr and Trp.

Particularly preferred conservative substitutions are as follows: Ala is substituted with Gly or Ser; Arg is substituted with Lys; Asn is substituted with Gin or His; Asp is substituted with Glu; Cys is substituted with Ser; Gln is substituted with Asn; Glu is substituted with Asp; Gly is substituted with Ala or Pro; His is substituted with Asn or Gln; Ile is substituted with Leu or Val; Leu is substituted with Ile or Val; Lys is substituted with Arg, Gln or Glu; Met is substituted with Leu, Tyr or Ile; Phe is substituted with Met, Leu or Tyr; Ser is substituted with Thr; Thr is substituted with Ser; Trp is substituted with Tyr; Tyr is substituted with Trp; and/or Phe is substituted with Val, Ile or Leu.

The numbering of Fc amino acids follows the Kabat numbering. "Kabat numbering" refers to the numbering system described by Kabat et al., the contents of which are recorded in the United States Department of Health and Human Services, "Sequence of Proteins of Immunological Interest" (1983). Specific numbering is shown in the following table:
Numbering of Fc amino acids based on the Kabat numbering system

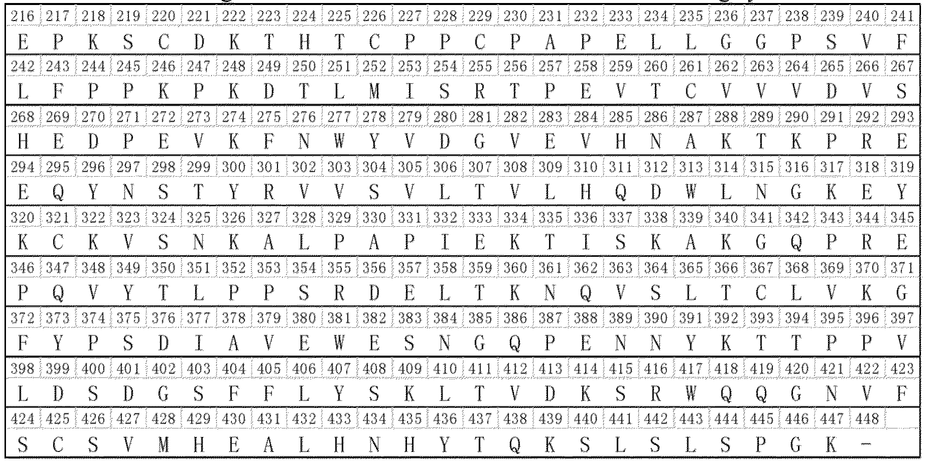

wherein
amino acids at positions 221-227 constitute the hinge domain,
amino acids at positions 228-340 constitute the second constant region CH2 domain of the heavy chain, and
amino acids at positions 341-447 constitute the third constant region CH3 domain of the heavy chain.

Antibodies can be modified to improve heterodimer pairing efficiency. For example, in certain aspects, compared to a wild-type antibody fragment, the Fc fragment of the monovalent unit heavy chain and/or the Fc fragment of the fusion peptide may comprise one or more substitutions that form a knob-into-hole structure pair therebetween. The knob-into-hole configuration is known in the art. See, e.g., Ridgway et al., "'Knob-into-holes' engineering of antibody CH3 domains for heavy chain heterodimerization," Protein Engineering 9(7):617-21 (1996).

In one aspect, T366 on one CH3 domain is substituted with a relatively larger amino acid residue, such as tyrosine (Y) or tryptophan (W), and Y407 on the other CH3 domain may then be substituted with a relatively smaller amino acid residue, such as threonine (T), alanine (A), or valine (V).

**Table 6. Combinations of Fc amino acid substitutions that form knob-into-hole structures between monovalent and single-chain units to improve heterodimer pairing efficiency**

| **Combination No.** | **Substitution on one CH3** | **Substitution on the other CH3** |
|---|---|---|
| 1 | T366W | Y407A |
| 2 | T366W | Y407V |
| 3 | T366Y | Y407A |
| 4 | T366Y | Y407V |
| 5 | T366W | T366S, L368A, Y407V |

In one aspect, one of the CH3 domains comprises one or more substitutions with an amino acid residue that is positively charged under physiological conditions, while the other CH3 domain comprises one or more substitutions with one or more amino acid residues that are negatively charged under physiological conditions. In one aspect, the positively charged amino acid residue may be arginine (R), histidine (H), or lysine (K). In another aspect, the negatively charged amino acid residue may be aspartic acid (D) or glutamic acid (E). Amino acid residues that may be substituted include, but are not limited to, D356, L368, K392, D399, and K409.

**Table 7. Combinations of CH3 amino acid substitutions that form ionic bonds between monovalent and single-chain units to improve heterodimer pairing efficiency**

| **Combination No.** | **Substitution on one CH3** | **Substitution on the other CH3** |
|---|---|---|
| 1 | D356K D399K | K392D K409D |
| 2 | L368R D399K | K392D K409D |
| 3 | L368K D399K | K392D K409D |
| 4 | L368R D399K | K409D |
| 5 | L368K D399K | K409D |
| 6 | L368R | K409D |
| 7 | L368K | K409D |

In one aspect, S354 on one CH3 domain is substituted with cysteine and Y349 on the other CH3 domain is also substituted with cysteine, with the residues at the two substituted positions forming a disulfide bond.

**Table 8. Combination of CH3 amino acid substitutions that forms a disulfide bond between monovalent and single-chain units to improve heterodimer pairing efficiency**

| **Combination No.** | **Substitution on one CH3** | **Substitution on the other CH3** |
|---|---|---|
| 1 | S354C | Y349C |

In one aspect, H435 and Y436 on one CH3 domain are substituted with arginine and phenylalanine, respectively, which leads to a significant decrease in binding ability between the Fc and protein A, thereby resulting in different protein A binding activities between the heterodimer and homodimer, making it easy to separate the two during affinity chromatography.

**Table 9. Amino acid substitutions of one CH3 that result in reduced binding ability to protein A**

| **Combination No.** | **Substitutions on CH3** |
|---|---|
| 1 | H435R, Y436F |

In a preferred embodiment of the present invention, the amino acid sequence of CH3 of the Fc forming the heterodimer is shown in the following table:

| **Amino acid sequence of CH3a** | **SEQ ID NO:** | **Amino acid sequence of CH3b** | **SEQ ID NO:** |
|---|---|---|---|
| GQPREPQVYTLPPSRDELTKNQVSLWCL VKGFYPSDIAVEWESNGQPENNYKTTPP VLDSDGSFFLYSKLTVDKSRWQQGNVFS CSVMHEALHNHYTQKSLSLSPGK | 72 | GQPREPQVYTLPPSRDELTKNQVSLSCAV KGFYPSDIAVEWESNGQPENNYKTTPPVL DSDGSFFLVSKLTVDKSRWQQGNVFSCSV MHEALHNHYTQKSLSLSPGK | 74 |
| GQPREPQVYTLPPCRDELTKNQVSLWCL VKGFYPSDIAVEWESNGQPENNYKTTPP VLDSDGSFFLYSKLTVDKSRWQQGNVFS CSVMHEALHNHYTQKSLSLSPGK | 9 | GQPREPQVCTLPPSRDELTKNQVSLSCAV KGFYPSDIAVEWESNGQPENNYKTTPPVL DSDGSFFLVSKLTVDKSRWQQGNVFSCSV MHEALHNHYTQKSLSLSPGK | 12 |
| GQPREPQVYTLPPCRDELTKNQVSLWCL VKGFYPSDIAVEWESNGQPENNYKTTPP VLDSDGSFFLYSKLTVDKSRWQQGNVFS CSVMHEALHNHYTQKSLSLSPGK | 9 | GQPREPQVCTLPPSRDELTKNQVSLSCAV KGFYPSDIAVEWESNGQPENNYKTTPPVL DSDGSFFLVSKLTVDKSRWQQGNVFSCSV MHEALHNRFTQKSLSLSPGK | 75 |
| GQPREPQVYTLPPSRDELTKNQVSLWCL VKGFYPSDIAVEWESNGQPENNYDTTPP VLDSDGSFFLYSDLTVDKSRWQQGNVFS CSVMHEALHNHYTQKSLSLSPGK | 8 | GQPREPQVYTLPPSRDELTKNQVSLTCRV KGFYPSDIAVEWESNGQPENNYKTTPPVL KSDGSFFLASKLTVDKSRWQQGNVFSCSV MHEALHNHYTQKSLSLSPGK | 11 |
| GQPREPQVYTLPPSRDELTKNQVSLTCLV KGFYPSDIAVEWESNGQPENNYDTTPPV LDSDGSFFLYSDLTVDKSRWQQGNVFSC SVMHEALHNHYTQKSLSLSPGK | 73 | GQPREPQVYTLPPSRKELTKNQVSLTCLVK GFYPSDIAVEWESNGQPENNYKTTPPVLK SDGSFFLYSKLTVDKSRWQQGNVFSCSVM HEALHNHYTQKSLSLSPGK | 76 |
| GQPREPQVYTLPPSRDELTKNQVSLSCA VKGFYPSDIAVEWESNGQPENNYKTTPP VLDSDGSFFLVSKLTVDKSRWQQGNVFS CSVMHEALHNHYTQKSLSLSPGK | 74 | GQPREPQVYTLPPSRDELTKNQVSLWCLV KGFYPSDIAVEWESNGQPENNYKTTPPVL DSDGSFFLYSKLTVDKSRWQQGNVFSCSV MHEALHNHYTQKSLSLSPGK | 72 |
| GQPREPQVCTLPPSRDELTKNQVSLSCA VKGFYPSDIAVEWESNGQPENNYKTTPP | 12 | GQPREPQVYTLPPCRDELTKNQVSLWCLV KGFYPSDIAVEWESNGQPENNYKTTPPVL | 9 |
| VLDSDGSFFL VSKLTVDKSRWQQGNVF SCSVMHEALHNHYTQKSLSLSPGK | | DSDGSFFLYSKLTVDKSRWQQGNVFSCSV MHEALHNHYTQKSLSLSPGK | |
| GQPREPQVCTLPPSRDELTKNQVSLSCA VKGFYPSDIAVEWESNGQPENNYKTTPP VLDSDGSFFLVSKLTVDKSRWQQGNVF SCSVMHEALHNRFTQKSLSLSPGK | 75 | GQPREPQVYTLPPCRDELTKNQVSLWCLV KGFYPSDIAVEWESNGQPENNYKTTPPVL DSDGSFFLYSKLTVDKSRWQQGNVFSCSV MHEALHNHYTQKSLSLSPGK | 9 |
| GQPREPQVYTLPPSRDELTKNQVSLTCR VKGFYPSDIAVEWESNGQPENNYKTTPP VLKSDGSFFLASKLTVDKSRWQQGNVFS CSVMHEALHNHYTQKSLSLSPGK | 11 | GQPREPQVYTLPPSRDELTKNQVSLWCLV KGFYPSDIAVEWESNGQPENNYDTTPPVL DSDGSFFLYSDLTVDKSRWQQGNVFSCSV MHEALHNHYTQKSLSLSPGK | 8 |
| GQPREPQVYTLPPSRKELTKNQVSLTCLV KGFYPSDIAVEWESNGQPENNYKTTPPV LKSDGSFFLYSKLTVDKSRWQQGNVFSC SVMHEALHNHYTQKSLSLSPGK | 76 | GQPREPQVYTLPPSRDELTKNQVSLTCLVK GFYPSDIAVEWESNGQPENNYDTTPPVLD SDGSFFLYSDLTVDKSRWQQGNVFSCSVM HEALHNHYTQKSLSLSPGK | 73 |

One embodiment of the present application provides a heterodimer antibody comprising two different antigen-binding polypeptide units. In certain aspects, the heterodimer differs in size from its corresponding homodimer, and the difference in size can be used to facilitate the separation of the heterodimer from the homodimer.

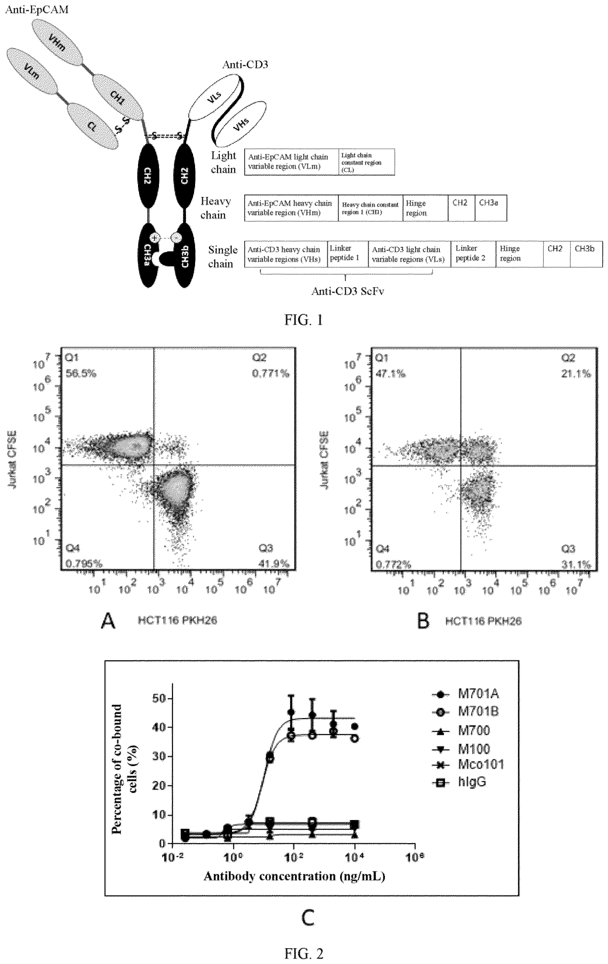

In certain aspects, as shown in FIG. 1, one of the two antigen-binding polypeptide units comprises a light chain-heavy chain pair similar to that of a wild-type antibody. Throughout the present application, this unit is also referred to as a "monovalent unit". In certain aspects, as shown in FIG. 1, the other antigen-binding polypeptide unit comprises a single-chain variable fragment (ScFv). Such an ScFv can be fused to the N-terminus of the constant fragment (Fc) of the antibody, referred to as a fusion peptide. Throughout the present application, this fusion peptide is also referred to as a "single-chain unit".

Any of the above antibodies or polypeptides may also comprise additional polypeptides, e.g., the peptides encoded as described herein, a signal peptide of the antibody constant region, which is used to direct secretion, or other heterologous polypeptides as described herein. The antibodies described herein may be modified such that their amino acid sequences are different from naturally occurring binding polypeptides from which they are derived. For example, a polypeptide or amino acid sequence from a given protein can be similar to the starting sequence, for example, can have a certain percentage of identity, such as 60%, 70%, 75%, 80%, 85%, 90%, 95%, 98% or 99% identity, to the starting sequence. In addition, nucleotide or amino acid substitutions, deletions or insertions may be made to effect conservative substitutions or alterations in "non-essential" amino acid regions. For example, a polypeptide or amino acid sequence from a given protein may be identical to the starting sequence, except for one or more independent amino acid substitutions, insertions or deletions, such as 1, 2, 3, 4, 5, 6, 7, 8, 9, 10, 15, 20, or more independent amino acid substitutions, insertions or deletions. In certain embodiments, a polypeptide or amino acid sequence from a given protein has 1 to 5, 1 to 10, 1 to 15, or 1 to 20 independent amino acid substitutions, insertions or deletions relative to the starting sequence.

As used herein, the term "detectable label" refers to a compound or composition that is directly or indirectly detectable, which directly or indirectly binds to a composition (e.g., a polynucleotide or a protein such as an antibody) to be detected to obtain a "labeled" composition. The term also includes sequences binding to the polynucleotide that provide signals through expression of the inserted sequences, such as green fluorescent protein (GFP) and the like. The label may itself be detectable (e.g., a radioisotope label or a fluorescent label) or, in the case of an enzyme label, may catalyze a detectable chemical change in the substrate compound or composition. The label can be used for small-scale detection or is more suitable for high-throughput screening. Likewise, suitable labels include, but are not limited to, radioisotopes, fluorescent dyes, chemiluminescent compounds, dyes, and proteins (including enzymes). The label is merely detectable or is also quantifiable. Reactions that are merely detectable typically include reactions whose presence can only be confirmed, while reactions that are quantifiable typically include reactions having quantifiable (e.g., numerically reportable) values such as intensity, polarization, and/or other properties. In luminescence or fluorescence analysis, a detectable reaction may directly use a luminophore or fluorophore that is associated with the analytical component and actually involves binding, or may indirectly use a luminophore or fluorophore that is linked to another component (e.g., a reporter molecule or an indicator).

In some embodiments, the antibody of the present invention may be conjugated with a therapeutic agent (e.g., a chemotherapeutic agent such as cisplatin and carboplatin), a prodrug, a peptide, a protein, an enzyme, a virus, a lipid, a biological response modifier, a pharmaceutical agent or PEG The antibody of the present invention can be linked or fused to a therapeutic agent, and the therapeutic agent may comprise a detectable label such as a radioactive label, an immunomodulator, a hormone, an enzyme, an oligonucleotide, a photoactive therapeutic or diagnostic agent, a cytotoxic agent that can be a drug or a toxin, an ultrasound enhancing agent, a non-radioactive label, a combination thereof, and other such ingredients known in the art.

In certain embodiments, the antigen-binding polypeptide comprises an amino acid sequence or one or more groups that do not normally bind to the antibody. For example, the single-chain Fv antibody fragment of the present application may comprise a flexible linker sequence, or may be modified to add a functional group (e.g., polyethylene glycol (PEG), a drug, a toxin, or a label). The antibody and the variants or derivatives thereof of the present application include modified derivatives, i.e., any type of molecule is covalently linked to the antibody and this covalent linkage does not prevent the antibody from binding to an epitope. In addition, the antibody may comprise one or more non-canonical amino acids.

It should be noted that an entity qualifier with unspecified quantity should be referred to as one or more (types of) that entity; for example, "multifunctional antibody" should be understood to mean one or more (types of) multifunctional antibodies. Likewise, the terms "one or more" and "at least one", which do not specify an exact number, are used interchangeably herein.

As used herein, the term "treatment" refers to both therapeutic treatments and prophylactic or preventative measures, wherein a subject is subjected to prevention or slowing down (alleviation) of an undesirable physiological change or disease, such as the development of cancer. Beneficial or desired clinical results include, but are not limited to, alleviation of symptoms, reduction in the severity of the disease, stabilization (e.g., prevention of worsening) of the state of the disease, delaying or slowing of the progression of the disease, amelioration or palliation of the state of the disease, and remission (whether partial or complete), whether detectable or undetectable. "Treatment" may also refer to prolonging the survival compared to the expected survival without the treatment. Conditions that require treatment include those already with the disorder or symptom, as well as those susceptible to the disorder or symptom or those in whom the disorder or symptom is to be prevented.

By "subject" or "individual" or "animal" or "patient" or "mammal" is meant any subject, particularly mammalian subjects, in need of diagnosis, prognosis or treatment. Mammalian subjects include humans, domestic animals, farm animals, zoos, sports grounds, or pets such as dogs, cats, guinea pigs, rabbits, rats, mice, horses, cattle, cows, primates (e.g., humans, monkeys such as cynomolgus monkeys, macaques, baboons, and chimpanzees, etc.), and the like.

As described herein, the antigen-binding polypeptides, variants or derivatives of the present application can be used in certain therapeutic and diagnostic methods related to cancer or infectious diseases. The present application also relates to an antibody-based therapy, comprising administering the bispecific antibody of the present application to a patient, such as an animal, a mammal and a human, for treating one or more diseases or conditions described herein. Therapeutic agents of the present application include, but are not limited to, the antibody of the present application (including the variants and derivatives thereof as described herein) and the nucleic acids or the polynucleotide encoding the antibody of the present application (including the variants and derivatives thereof as described herein). The antibody of the present application may also be used to treat, inhibit or prevent a disease, disorder or condition, including a malignant disease or disorder, or a condition associated with such a disease or disorder, such as a disease associated with an immune response. In some embodiments, the antibody of the invention may be used as an immunosuppressant. In some embodiments, the antibody of the invention may be used to treat an autoimmune disease. The antigen-binding polypeptides of the present application and variants or derivatives thereof are useful for inhibiting the growth, development and/or metastasis of cancer, particularly those listed above or in the following paragraphs.

Other diseases or conditions associated with increased cell survival that can be treated, prevented, diagnosed and/or predicted with the antibody or the variants or derivatives thereof of the present application include, but are not limited to, cancer or tumors, including the development and/or metastasis of malignant tumors, as well as related diseases (e.g., malignant ascites, malignant pleural effusion, and malignant effusion), such as EpCAM-positive tumors.

Methods for administering the antibody or the variants or derivatives thereof include, but are not limited to, intradermal, intramuscular, intraperitoneal, intravenous, subcutaneous, intranasal, epidural and oral routes. The antibody or composition may be administered by any convenient route, for example, by infusion or bolus injection, or by absorption through the epithelium or mucosa and the inner layers of the skin (e.g., oral mucosa, rectal and intestinal mucosa, etc.), and may be administered with other biologically active agents. Thus, the pharmaceutical composition comprising the antibody of the present application may be administered orally, rectally, parenterally, intracisternally, intravaginally, intraperitoneally, topically (e.g., via powder, ointment, drop or transdermal patch), buccally, or as an oral or nasal spray. As used herein, the term "parenteral" refers to routes of administration that include intravenous, intramuscular, intraperitoneal, intrasternal, subcutaneous and intraarticular injections and infusions. Th administration may be systemic or local. It may also be desirable to locally administer the antigen-binding polypeptide or composition of the present application to the area in need of treatment; this can be achieved by, for example but not limited to, local perfusion during surgery, local application, for example, in combination with a post-operative wound dressing, by injection, through a catheter, by a suppository, or by an implant, wherein the implant is a porous, non-porous, or gelatinous material, including membranes or fibers. Preferably, when administering the proteins (including the antibody) of the present application, care must be taken to use materials that do not absorb the proteins.

### BRIEF DESCRIPTION OF THE DRAWINGS

FIG. 1 shows a schematic structural diagram of a YBODY antibody.
FIG. 2 shows the detection of the binding interaction between HCT116 and Jurkat cells mediated by bispecific antibodies. A: a flow cytometry graph for a negative control (HCT116 + Jurkat without antibody), where Q1 is for CFSE-stained Jurkat cells, Q2 is for co-bound Jurkat and HCT116 cells, Q3 is for PKH26-stained HCT116 cells, and Q4 is for unstained cells; B: a flow cytometry graph for an experimental group (HCT116 + Jurkat + M701A, 10 µg/mL), wherein the representation meaning of each quadrant is the same as that in the previous graph; and C: concentration gradient curves for the binding of HCT116 to Jurkat cells mediated by different antibodies.
FIG. 3 shows the detection of the biological activity of bispecific antibodies (reporter gene system).
FIG. 4 shows the detection of the *in-vitro* killing effect mediated by bispecific antibodies. A: *in-vitro* killing of B16-EpCAM by M701A; B: *in-vitro* killing of B16 by M701A; C: *in-vitro* killing of HCT116 by M701A; D: *in-vitro* killing of OVCAR-3 by M701A; E: *in-vitro* killing of CHO-K1-huEpCAM by different bispecific antibodies; and F: *in-vitro* killing of HCT116 by different bispecific antibodies.
FIG. 5 shows the *in-vivo* efficacy of bispecific antibodies in an HCT116 human colon cancer model. A: changes in the growth of mouse tumor volume; and B: changes in mouse body weight.
FIG. 6 shows the *in-vivo* efficacy of bispecific antibodies in an OVCAR-3 human ovarian cancer model. A: changes in the growth of mouse tumor volume; and B: changes in mouse body weight.

### DETAILED DESCRIPTION

The method and use of the present invention are described below with reference to the drawings, and the examples given are for illustrative purposes only and are not intended to limit the scope of the present invention. For those of ordinary skills in the art to which the present invention pertains, various simple deductions or substitutions may be made without departing from the spirit of the present invention, and should be construed as falling within the scope of the present invention.

### Example 1: Construction of Expression Vectors of Bispecific Antibodies

Each bispecific antibody structure targeting EpCAM and CD3 contained an anti-EpCAM binding region and an anti-CD3 binding region, wherein the monovalent unit was a pair formed by anti-EpCAM heavy and light chains, and the single-chain unit was an anti-CD3 ScFv-Fc form. The structure was defined as a YBODY structure (as shown in FIG. 1), wherein the anti-CD3 VLs are linked to the hinge region and CH2 via a linker. In the structure, the heavy chain Fc of the monovalent unit and the Fc of the single-chain unit (with human IgG heavy chain Fc as the backbone) were subjected to amino acid mutation modifications, making them less prone to form homodimers and more prone to form heterodimers. Using an existing plasmid or synthetic gene fragment as a template, each chain corresponding to the bispecific antibody was amplified through PCR and overlap PCR, and each chain of the antibody was cloned into a pcDNA3.1 vector (Invitrogen) by means of enzyme digestion and ligation or recombination methods. Specific sequence information of each chain of the antibody is shown in Table 1 and the sequence listing.

**Table 1. Amino acid sequence information corresponding to each antibody molecule**

| Bispecific antibody No. | Anti-EpCAM Light chain SEQ ID NO: | | Anti-EpCAM Heavy chain SEQ ID NO: | | | | | Anti-CD3 Single chain SEQ ID NO: | | | | |
|---|---|---|---|---|---|---|---|---|---|---|---|---|
| | VL | CL | VH | CH1 | Hinge | CH2 | CH3a | ScFv | L2 | Hinge | CH2 | CH3b |
| M701 | 13 | 1 | 14 | 2 | 3 | 6 | 8 | 17 | 5 | 3 | 6 | 10 |
| M701A | 13 | | 14 | | | 6 | 8 | 18 | | | 6 | 11 |
| M701B | 13 | | 14 | | | 6 | 8 | 19 | | | 6 | 11 |
| M701C | 13 | | 14 | | | 6 | 8 | 20 | | | 6 | 11 |
| M701D | 13 | | 14 | | | 6 | 8 | 21 | | | 6 | 11 |
| M701E | 13 | | 14 | | | 6 | 8 | 22 | | | 6 | 11 |
| M701F | 13 | | 14 | | | 6 | 8 | 23 | | | 6 | 11 |
| M701G | 13 | | 14 | | | 6 | 8 | 24 | | | 6 | 11 |
| M701H | 15 | | 16 | | | 6 | 8 | 18 | | | 6 | 11 |
| M701I | 15 | | 16 | | | 6 | 8 | 19 | | | 6 | 11 |
| M701J | 13 | | 14 | | | 7 | 9 | 18 | | | 7 | 12 |
| M701K | 13 | | 14 | | | 7 | 9 | 19 | | | 7 | 12 |

### Example 2: Expression and Purification of Bispecific Antibodies

The plasmid was extracted according to a conventional plasmid extraction method and used for chemical transfection of CHO-S cells (from Gibco). The transfected cells were cultured in a shaker with 5% CO₂ at 37 °C for 7-10 days. The supernatant was collected by centrifugation at 3000× g, filtered through a 0.22 µm filter, and subjected to protein A affinity chromatography to obtain the preliminarily purified bispecific antibody. The concentration of the purified protein was determined by the UV absorbance at 280 nm and the corresponding extinction coefficient, the purity of the antibody was tested by high-performance size exclusion chromatography (HPLC-SEC), and the expression level corresponding to each protein was calculated. The bispecific antibody molecules showed expression levels ranging from 40 mg/L to 91 mg/L and initial purities ranging from 45% to 81%, and the expression levels and initial purities of M701A, M701B, M701C, M701D, M701E, M701F, M701G, M701H, M701I, M701J, and M701K were significantly superior to those of M701. Next, the affinity samples were further purified by cation exchange chromatography to finally obtain bispecific antibodies with an HPLC-SEC purity of >95%. The purification recovery rates for the bispecific antibodies are shown in Table 2.

**Table 2. Expression levels and initial HPLC-SEC purities of bispecific antibodies**

| Antibody No. | Expression level | HPLC-SEC | Purification recovery rate |
|---|---|---|---|
| M701 | 47.2mg/L | 45.91% | 14% |
| M701A | 90.8 mg/L | 81.12% | 57% |
| M701B | 84.1 mg/L | 78.53% | 53% |
| M701C | 54.4 mg/L | 64.54% | 32% |
| M701D | 65.8 mg/L | 58.63% | 23% |
| M701E | 49 mg/L | 55.76% | 22% |
| M701F | 52.4 mg/L | 66.32% | 30% |
| M701G | 41 mg/L | 70.76% | 25% |
| M701H | 73.3 mg/L | 72.03% | 43% |
| M701I | 80.6mg/L | 73.21% | 45% |
| M701J | 87.7 mg/L | 78.21% | 47% |
| M701K | 81.3mg/L | 74.50% | 41% |

### Example 3: Thermal Stability Assay for Bispecific Antibodies

A fine sample of each bispecific antibody was diluted to 0.5 mg/mL with a buffer (25 mM citric acid + 50 mM NaCl, pH 6.0) and aliquoted into 1.5 mL EP tubes at 100 µL/tube. The aliquots were placed in a 40 °C water bath for a 14-day thermal acceleration experiment and assayed for changes in purity and affinity. The day the aliquots were placed in the 40 °C water bath was designated as D0, with the 14th day designated as D14.

A human EpCAM antigen (SB, Cat: 10694-H08H) and a human CD3 antigen (SB, Cat: CT038-H2508H) were separately immobilized on a CM5 chip using an amino coupling method, with an antigen coupling level of 1500 RU. For the detection of the binding activity to antigens, samples were diluted to the initial concentration with 1× HBS-EP + buffer, followed by 2-fold gradient dilution to achieve 4 concentrations. The samples were then detected on a machine from a low concentration to a high concentration, with a binding flow rate of 30 µL/min, a binding time of 120 s, and a dissociation time of 300 s. The chip was regenerated using a Glycine solution at pH 1.5, with a regeneration flow rate of 10 µL/min and a regeneration time of 30 s. After the detection was completed, data fitting was carried out on the result using the Biacore T200 Evaluation Software in a 1:1 binding fitting mode to obtain an equilibrium dissociation constant (KD).

The results, as shown in Table 3, indicate that M701A, M701B, M701H, M701I, M701J, and M701K exhibited a decrease in purity of less than 5% on the 14th day of the accelerated thermal testing, wherein M701A, M701B, M701J, and M701K exhibited a change in purity of less than 2%, with the affinity for both antigens substantially unchanged, suggesting that M701A, M701B, M701J, and M701K had good thermal stability.

**Table 3. Purity and affinity assays for thermally accelerated samples of bispecific antibodies**

| Antibody No. | HPLC-SEC% | | EpCAM-Biacore (K_{D}, nM) | | CD3-Biacore (K_{D}, nM) | |
|---|---|---|---|---|---|---|
| | D0 | D14 | D0 | D14 | D0 | D14 |
| M701 | 95.21% | 25.11% | 19.17 | n.d. | 25.44 | n.d. |
| M701A | 95.80% | 95.78% | 17.11 | 17.28 | 21.15 | 21.07 |
| M701B | 95.63% | 94.26% | 20.01 | 22.61 | 28.27 | 31.52 |
| M701C | 95.34% | 85.10% | 18.57 | n.d. | 37.20 | n.d. |
| M701D | 95.87% | 86.78% | 14.39 | n.d. | 51.73 | n.d. |
| M701E | 96.03% | 89.33% | 23.14 | n.d. | 31.46 | n.d. |
| M701F | 95.48% | 84.48% | 17.94 | n.d. | 61.34 | n.d. |
| M701G | 95.22% | 80.22% | 19.71 | n.d. | 18.80 | n.d. |
| M701H | 95.07% | 90.72% | 51.56 | 60.91 | 95.26 | 100.4 |
| M701I | 95.57% | 91.27% | 49.78 | 57.05 | 40.03 | 45.56 |
| M701J | 95.60% | 95.23% | 18.23 | 18.67 | 23.45 | 23.56 |
| M701K | 95.05% | 94.56% | 21.05 | 22.58 | 28.78 | 30.63 |

| | | | | | | |
|---|---|---|---|---|---|---|
| Note: n.d. indicates not detected. | | | | | | |

### Example 4: Detection of Affinity of Bispecific Antibody for EpCAM of Cells

The affinity of the antibodies for human EpCAM on the cell surface was assayed by FACS using human colon cancer cells HCT116 (Shanghai Institutes for Biological Sciences) as positive cells expressing human EpCAM on the cell membrane surface.

HCT116 cells were collected by centrifugation, resuspended in a buffer (PBS + 1% FBS), and added to a 96-well plate at 2×10⁵ cells/well (50 µL/well). Then the cells were centrifuged at 350× g for 5 min, and the supernatant was removed. Each bispecific antibody was diluted to 1000 nM with buffer, subjected to serial gradient dilution, and then added to the 96-well plate at 50 µL/well. The mixture was resuspended, then incubated for 1 h in the dark, and centrifuged. The supernatant was then removed, and the wells were washed twice with buffer, followed by resuspension in a diluted PE-labeled anti-human IgG Fc antibody (Biolegend, 409304). The suspension was then incubated for 30 min in the dark, washed twice with buffer, then resuspended in 100 µL of buffer, and detected on a flow cytometer (BD Accuri^{™} C6).

The bispecific antibodies all showed significant binding to HCT116 cells, among which M700 served as an anti-EpCAM monoclonal antibody control (with a light chain of SEQ ID NO: 25 and a heavy chain of SEQ ID NO: 26), with specific EC50 values as shown in Table 4.

**Table 4. Binding ability of bispecific antibodies to HCT116 cells**

| Antibody No. | M700 | M701 | M701A | M701B | M701H | M701I | M701J | M701K |
|---|---|---|---|---|---|---|---|---|
| EC50 (nM) | 1.782 | 10.501 | 3.565 | 3.719 | 7.151 | 7.086 | 3.779 | 5.652 |

### Example 5: Detection of Affinity of Bispecific Antibody for CD3 (Biacore)

A human CD3 antigen (SB, Cat: CT038-H2508H) was immobilized on a CM5 chip using an amino coupling method, with an antigen coupling level of 1500 RU. For the detection of the binding activity to the CD3 antigen, samples were diluted to the initial concentration with 1× HBS-EP + buffer, followed by 2-fold gradient dilution to achieve 4 concentrations. The samples were then detected on a machine from a low concentration to a high concentration, with a binding flow rate of 30 µL/min, a binding time of 120 s, and a dissociation time of 300 s. The chip was regenerated using a Glycine solution at pH 1.5, with a regeneration flow rate of 10 µL/min and a regeneration time of 30 s. After the detection was completed, data fitting was carried out on the result using the Biacore T200 Evaluation Software in a 1:1 binding fitting mode to obtain an equilibrium dissociation constant (KD). As shown in Table 5, the series of bispecific antibodies all showed binding to the human CD3 antigen. Among them, M701A and M701B have identical anti-EpCAM antibody variable region sequences (SEQ ID NO: 13 and SEQ ID NO: 14), and their anti-CD3 antibody variable region sequences are SEQ ID NO: 18 and SEQ ID NO: 19, respectively, with corresponding affinities of 21.15 nM and 28.27 nM, respectively. However, the anti-EpCAM antibody variable region sequences were changed to SEQ ID NO: 15 and SEQ ID NO: 16, while the anti-CD3 antibody variable region sequences were still SEQ ID NO: 18 and SEQ ID NO: 19, and the affinities of the corresponding bispecific antibodies M701H and M701I were 95.26 nM and 40.03 nM, respectively. This indicates that the combinations of different anti-EpCAM antibodies with different anti-CD3 antibodies to form bispecific antibodies exhibited irregular affinity performance.

**Table 5. Detection of binding ability of bispecific antibodies to human CD3 antigen by Biacore**

| Antibody No. | M701 | M701A | M701B | M701H | M701I | M701J | M701K |
|---|---|---|---|---|---|---|---|
| Kd (nM) | 25.44 | 21.15 | 28.27 | 95.26 | 40.03 | 23.45 | 28.78 |

### Example 6: Bispecific Antibody-Mediated Cell Bridging

EpCAM-positive cell line HCT116 was stained with PKH26, and CD3-positive cell line Jurkat (Shanghai Institutes for Biological Sciences) was stained with CFSE. The stained cells were mixed and co-incubated at a ratio of 1:1 (1×10⁵ HCT116 cells: 1×10⁵ Jurkat cells) with test antibodies subjected to gradient dilution, among which M700 served as an anti-EpCAM monoclonal antibody control (with a light chain of SEQ ID NO: 25 and a heavy chain of SEQ ID NO: 26), M100 served as an anti-CD3 monoclonal antibody control (with a light chain of SEQ ID NO: 27 and a heavy chain of SEQ ID NO: 28), and Mco101 was an anti-luciferase and anti-CD3 bispecific antibody and served as a bispecific antibody anti-CD3 isotype control (with a light chain of SEQ ID NO: 29, a heavy chain of SEQ ID NO: 30 and a single chain of SEQ ID NO: 31, structurally identical to FIG. 1). After 1 h of co-incubation, the mixtures were washed, resuspended, and detected on a flow cytometer (BD Accuri^{™} C6). The cells that are double positive for CFSE and PKH26 are the HCT116 and Jurkat cells bridged by the antibody.

The results, as shown in FIG. 2, indicate that after the Jurkat and HCT116 cells were mixed at a ratio of 1:1 and co-incubated with hIgG, M700, M100, and Mco101 for 1 h, the HCT116 and Jurkat cells were not bridged; however, after the addition of M701A and M701B, the bridged cells accounted for about 40% of the total PKH26-positive cells, M701A and M701B had comparable action activity, and the bispecific antibody-mediated cell interaction demonstrated a positive dose-effect relationship with the antibody concentration.

### Example 7: Detection of Biological Activity of Bispecific Antibodies (Reporter Gene Method)

The biological activity of the bispecific antibodies was detected using Jurkat-CD3-NFAT-RE-Luc cells (Promega). A pLV-puro (Inovogen Tech. Co., cat. No. VL3001) vector containing DNA encoding a human EpCAM gene (NCBI sequence No. NM_002354.3) was transfected into CHO-K1 cells to obtain a cell line CHO-K1-huEpCAM stably expressing human EpCAM. CHO-K1-huEpCAM cells were collected as target cells and resuspended in a buffer (PBS + 1% FBS), added to a white 96-well culture plate at 4×10⁴ cells/well, and incubated overnight in an incubator at 37 °C with 5% CO₂ for 18-24 h. The medium in the plate was then removed, and 40 µL of antibody dilution was added to each well. The Jurkat-CD3-NFAT-RE-Luc cells, which served as effector cells, were taken out, and pipetted to prepare a single-cell suspension. The suspension was added to a white 96-well culture plate at an effect-target (E:T) ratio of 1.5:1, with 40 µL per well, corresponding to 6×10⁴ cells per well. The white 96-well culture plate was incubated in an incubator at 37 °C with 5% CO₂ for 6 h, and then 80 µL of a Bio-Glo luciferase assay reagent was added to each well. The plate was incubated in the dark at room temperature for 15 min and then placed in a multifunctional plate reader, where luminescence values were read using chemiluminescence.

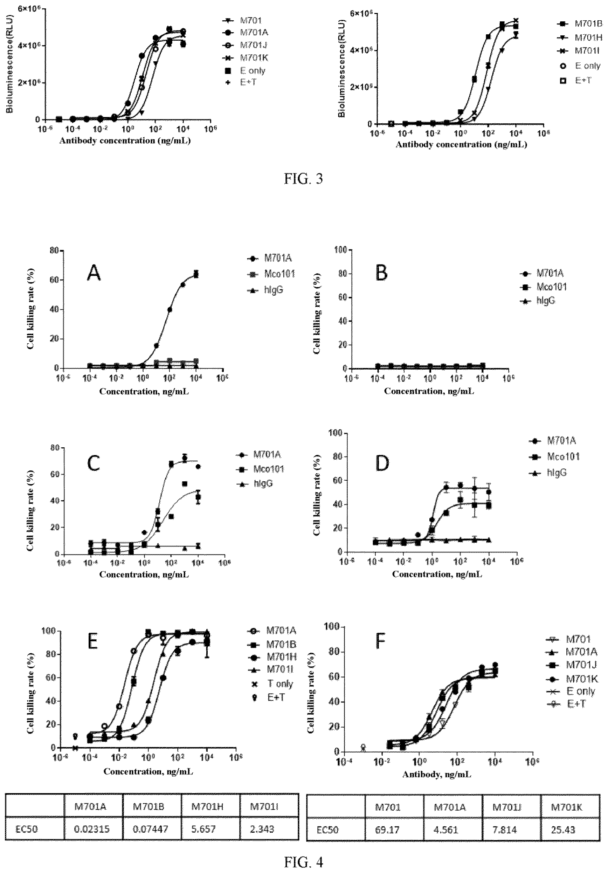

The results, as shown in FIG. 3, indicate that in the reporter gene evaluation system, all of the bispecific antibodies M701, M701A, M701B, M701H, M701I, M701J and M701K exhibited biological activity, with M701A, M701J, and M701K showing stronger activity than M701.

### Example 8: Detection of In- Vitro Killing Effect Mediated by Bispecific Antibodies

The *in-vitro* killing effect mediated by the bispecific antibodies was detected by using isolated PBMCs as effector cells and EpCAM-expressing cells as target cells. A pLV-puro (Inovogen Tech. Co., cat. No. VL3001) vector containing DNA encoding a human EpCAM gene (NCBI sequence No. NM_002354.3) was transfected into mouse melanoma cells B16 (Shanghai Institutes for Biological Sciences) to obtain a cell line B16-EpCAM stably expressing human EpCAM, wherein B16 cells served as negative cells not expressing EpCAM. Other EpCAM-expressing target cells also include human colon cancer cells HCT116 (Shanghai Institutes for Biological Sciences), human ovarian cancer cells OVCAR-3 (China Center for Type Culture Collection (CCTCC)), and CHO-K1-huEpCAM. The cells were digested with pancreatin to prepare a single-cell suspension, and the suspension was centrifuged at 300 g for 5 min to collect cells. The cells were stained with 5 µM 5,6-carboxyfluorescein diacetate, succinimidyl ester (CFSE) at 37 °C for 15 min, washed twice with a complete medium, then counted on a Vi-cell cell counter, and added to a 96-well plate at 2×10⁴ cells/100 µL per well as designed for the experiment. Each antibody at a corresponding concentration was added at 50 µL/well, and hPBMCs were counted on a Cellometer cell counter and then added to the 96-well plate (at 2×10⁵ cells/50 µL per well and with an effect-target ratio of 10:1). The cell culture plate was placed in a cell incubator for culturing for 72 h, and after the cells were digested to prepare a single cell suspension, a propidium iodide (PI) solution with a final concentration of 1 µg/mL was added. The cells were incubated for 10 min and then detected on a flow cytometer (BD Accuri^{™} C6), and the percentage of CFSE+PI+double positive cells in CFSE+positive cells was analyzed.

As shown in FIGs. 4A and 4B, M701A exhibited a killing effect only on B16-EpCAM cells expressing EpCAM, with no killing effect on B16 cells not expressing EpCAM, while the control antibody Mco101 did not exhibit a killing effect on any cells, indicating that the targeting effect of the bispecific antibody. As shown in FIGs. 4C and 4D, M701A exhibited a significant killing effect on both HCT116 cells and OVCAR-3 cells, and the effect was stronger than that of the control Mco101. As shown in FIG. 4E, the bispecific antibodies M701A, M701B, M701H and M701I all exhibited significant killing effects on CHO-K1-huEpCAM cells, and the effects of M701A and M701B were significantly stronger than those of M701H and M701I. As shown in FIG. 4F, the bispecific antibodies M701J and M701K both exhibited significant killing effects on HCT116 cells, with killing EC₅₀ values of 7.814-25.43 ng/mL, and the killing level of M701J was not significantly different from that of M701A. The killing activity of the above bispecific antibodies was significantly stronger than that of M701 (the EC₅₀ value for M701 was 69.17 ng/mL).

### Example 9: In-Vivo Efficacy of Bispecific Antibodies in HCT116 Human Colon Cancer Heterotopic Xenograft Tumor Model

Sufficient quantities of HCT116 cells and effector cells (cytokine-induced killer (CIK) cells, a heterogeneous population of cells obtained by co-culturing human peripheral blood mononuclear cells *in vitro* with various cytokines for a period of time; these cells express both CD3+ and CD56+ membrane protein molecules and are therefore also known as NK cell-like T lymphocytes) were cultured according to culture conditions, then collected, and counted. Each mouse was inoculated on the right dorsal side with pre-mixed HCT116 cells (2× 10⁶ cells/mouse) and CIK cells (2×10⁶ cells/mouse) in a volume of 0.1 mL/mouse to establish a human colon cancer HCT116 xenograft tumor model. Administration for treatment began 1 h after inoculation. The mice were divided into groups as follows: test drug M701A 2 mg/kg group, test drug M701A 1 mg/kg group, M701 1 mg/kg group, monoclonal antibody control M700 2 mg/kg group, and vehicle control group (normal saline), with 8 mice in each group. The administration was performed by tail vein injection on days 0, 2 and 4 after inoculation, for a total of three administrations. The therapeutic effects were evaluated according to the relative tumor inhibition rate and the complete tumor regression rate, and the safety was evaluated according to the changes in body weight and the mortality of the animals.

Tumor size calculation formula: tumor volume (mm³) = 0.5 × (long diameter of tumor × short diameter of tumor²).

**Relative tumor inhibition rate TGI (%):** TGI = 1 - T/C (%). T and C are the tumor volumes (TVs) at a particular time point for the treatment and control groups, respectively. The calculation formula is as follows: T/C% = T_{TV} / C_{TV} × 100% (T_{TV}: mean tumor volume for treatment group; C_{TV}: mean tumor volume for vehicle control group).

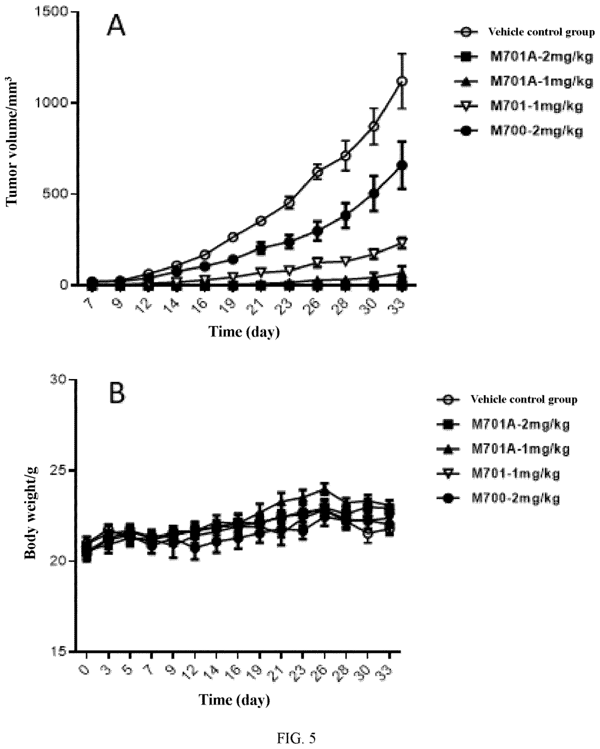

**Complete tumor regression:** defined as the condition in which the tumor volume decreases to below 63 mm³ during or after treatment. Complete tumor regression rate (%) = number of animals with complete regression in a group / total number of animals in that group × 100%. As shown in FIG. 5A, the test drug M701A (2 mg/kg, 1 mg/kg) exhibited a significant tumor inhibitory effect on day 30 after interruption of the administration (i.e., day 33 after inoculation) in the treatment groups, with relative tumor inhibition rates (TGI%) of 100% and 93.82%, respectively. Relative to the vehicle control group, all other groups exhibited statistically significant differences (p-values < 0.001), and in the two groups treated with M701A, all tumors in the 2 mg/kg group met the criteria for complete regression. The efficacy of M701A at both doses was significantly superior to that of M700 (p-values < 0.001) at 2 mg/kg, and the efficacy of M701A was significantly superior to that of M701 at the same dose (both at 1 mg/kg). As shown in FIG. 5B, there was no body weight loss in the animals during the treatment, and no signs of drug toxicity were observed.

In the same tumor model, M701B, M701J and M701K all exhibited similar tumor inhibitory effects to M701A at the same dose, with no body weight loss observed.

### Example 10: In-Vivo Efficacy of Bispecific Antibodies in OVCAR-3 Human Ovarian Cancer Heterotopic Xenograft Tumor Model

Sufficient quantities of OVCAR-3 cells and effector cells (CIK cells) were cultured according to culture conditions, then collected, and counted. Each mouse was inoculated on the right dorsal side with pre-mixed OVCAR-3 cells (1×10⁷ cells/mouse) and CIK cells (1×10⁷ cells/mouse) in an inoculation volume of 0.2 mL/mouse and with a Matrigel content of 50% (0.1 mL/mouse) to establish a human ovarian cancer OVCAR-3 heterotopic transplantation tumor model. Administration for treatment was performed 1 h after inoculation. The mice were divided into groups as follows: test drug M701A (5 mg/kg) group, anti-CD3 isotype control Mco101 (5 mg/kg) group, monoclonal antibody control M700 (5 mg/kg) group, and vehicle control group (normal saline), with 8 mice in each group. The administration was performed by tail vein injection on days 0, 2 and 4 after inoculation, for a total of three administrations. The therapeutic effects were evaluated according to the relative tumor inhibition rate (TGI) and the complete tumor regression rate, and the safety was evaluated according to the changes in body weight and the mortality of the animals.

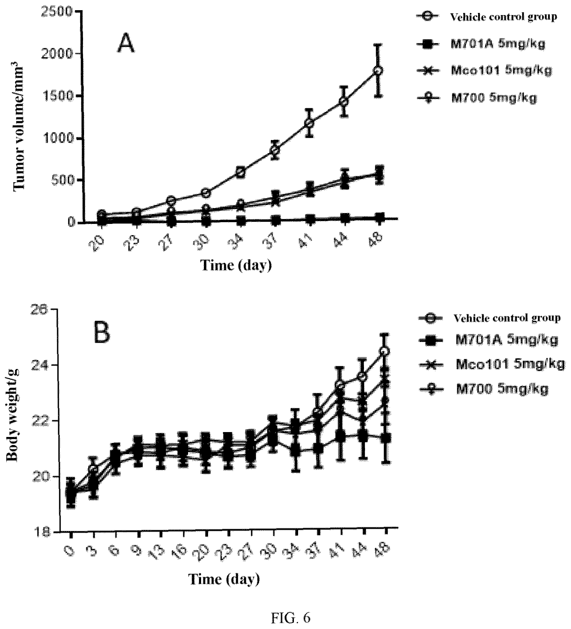

As shown in FIG. 6A, the test drug M701A (5 mg/kg) exhibited a significant tumor inhibitory effect on day 44 after interruption of the administration (i.e., day 48 after inoculation) in the treatment group, with a relative tumor inhibition rate (TGI%) of 98.97%. Relative to the vehicle control group, all other groups exhibited statistically significant differences (p-values < 0.001); in the M701A (5 mg/kg) group, the complete tumor regression rate was 87.5%, and the efficacy of this group was significantly superior to that of the M700 (5 mg/kg) group (TGI = 70.42%) (p-values < 0.001) and the Mco101 (5 mg/kg) group (TGI = 68.87%) (p-values < 0.001). As shown in FIG. 6B, there was no body weight loss in the animals during the treatment, and no signs of drug toxicity were observed.

In the same tumor model, M701B, M701J and M701K all exhibited similar tumor inhibitory effects to M701A at the same dose, with no body weight loss observed.

All documents mentioned in the present application are incorporated by reference, just as each document is cited separately as a reference. In addition, it should be understood that various modifications or changes may be made by those skilled in the art after reading the above teachings of the present application, and these equivalent forms also fall within the scope defined by the claims appended hereto.

| **SEQUENCE LISTING** | |
|---|---|
| Name | Amino acid sequence |
| Light chain constant region CL SEQ ID NO: 1 | |
| Light chain constant region CL SEQ ID NO: 60 | GQPKANPTVTLFPPSSEELQANKATLVCLISDFYPGAVTVAWKADGSPVKAGVE TTKPSKQSNNKYAASSYLSLTPEQWKSHRSYSCQVTHEGSTVEKTVAPTECS |
| Light chain constant region CL SEQ ID NO: 61 | GQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| Light chain constant region CL SEQ ID NO: 62 | GQPKAAPSVTLFPPSSEELQANKATLVCLISDFYPGAVTVAWKADSSPAKAGVE TTTPSKQSNNKYAASSYLSLTPEQWKSHKSYSCQVTHEGSTVEKTVAPTECS |
| Light chain constant region CL SEQ ID NO: 63 | GQPKAAPSVTLFPPSSEELQANKATLVCLISDFYPGAVKVAWKADGSPVNTGVE TTTPSKQSNNKYAASSYLSLTPEQWKSHRSYSCQVTHEGSTVEKTVAPAECS |
| Light chain constant region CL SEQ ID NO: 64 | GQPKAAPTVTLFPPSSEELQANKATLVCLISDFYPGAVKVAWKADSSPAKAGVE TTTPSKQSNNKYAASSYLSLTPEQWKSHKSYSCQVTHEGSTVEKTVAPTECS |
| Light chain constant region CL SEQ ID NO: 65 | GQPKANPTVTLFPPSSEELQANKATLVCLISDFYPGAVTVAWKADGSPVKAGVE TTKPSKQSNNKYAASSYLSLTPEQWKSHRSYSCQVTHEGSTVEKTVAPTECS |
| Heavy chain constant region CH1 SEQ ID NO: 2 | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKV |
| Hinge region SEQ ID NO: 3 | EPKSCDKTHTCPPCP |
| Linker peptide 1 (L1) SEQ ID NO: 4 | GGGGSGGGGSGGGGS |
| Linker peptide 2 (L2) SEQ ID NO: 5 | GAAA |
| Heavy chain constant region CH2 SEQ ID NO: 6 | APELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTIS KAK |
| Heavy chain constant region CH2 SEQ ID NO: 7 | APPVAGPSVFLFPPKPKDTLMISRTPEVTCVVVDVSHEAPEVQFNWYVDGVEVH NAKTKPREEQFNSTFRVVSVLTVVHQDWLNGKEYKCKVSNKGLPAPIEKTISKT K |
| Heavy chain constant region CH2 SEQ ID NO: 66 | APEAAGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALGAPIEKTI SKAK |
| Heavy chain constant region CH2 SEQ ID NO: 67 | APEFEGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPASIEKTIS KAK |
| Heavy chain constant region CH2 SEQ ID NO: 68 | APELLGGPSVFLFPPKPKDTLMISRTPEVTCVVVDVSHEDPEVKFNWYVDGVEV HNAKTKPREEQYASTYRVVSVLTVLHQDWLNGKEYKCKVSNKALPAPIEKTIS KAK |
| Heavy chain constant region CH2 SEQ ID NO: 69 | APELLGGPSVFLFPPKPKDTLMISRTPEVTCVVVDVSHEDPEVKFNWYVDGVEV HNAKTKPREEQYQSTYRVVSVLTVLHQDWLNGKEYKCKVSNKALPAPIEKTIS KAK |
| Heavy chain constant region CH2 SEQ ID NO: 70 | APEAAGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTI SKAK |
| Heavy chain constant | APPVAGPSVFLFPPKPKDTLMISRTPEVTCVVVDVSHEDPEVQFNWYVDGVEVH |
| region CH2 SEQ ID NO: 71 | NAKTKPREEQFNSTFRVVSVLTVVHQDWLNGKEYKCKVSNKGLPAPIEKTISKT K |
| Heavy chain constant region CH3 SEQ ID NO: 8 | GQPREPQVYTLPPSRDELTKNQVSLWCLVKGFYPSDIAVEWESNGQPENNYDT TPPVLDSDGSFFLYSDLTVDKSRWQQGNVFSCSVMHEALHNHYTQKSLSLSPG K |
| Heavy chain constant region CH3 SEQ ID NO: 9 | GQPREPQVYTLPPCRDELTKNQVSLWCLVKGFYPSDIAVEWESNGQPENNYKT TPPVLDSDGSFFLYSKLTVDKSRWQQGNVFSCSVMHEALHNHYTQKSLSLSPG K |
| Heavy chain constant region CH3 SEQ ID NO: 10 | GQPREPQVYTLPPSRDELTKNQVSLTCLVKGFYPSDIAVEWESNGQPENNYKTT PPVLDSDGSFFLYSKLTVDKSRWQQGNVFSCSVMHEALHNHYTQKSLSLSPGK |
| Heavy chain constant region CH3 SEQ ID NO: 11 | GQPREPQVYTLPPSRDELTKNQVSLTCRVKGFYPSDIAVEWESNGQPENNYKTT PPVLKSDGSFFLASKLTVDKSRWQQGNVFSCSVMHEALHNHYTQKSLSLSPGK |
| Heavy chain constant region CH3 SEQ ID NO: 12 | GQPREPQVCTLPPSRDELTKNQVSLSCAVKGFYPSDIAVEWESNGQPENNYKTT PPVLDSDGSFFLVSKLTVDKSRWQQGNVFSCSVMHEALHNHYTQKSLSLSPGK |
| Heavy chain constant region CH3 SEQ ID NO: 72 | GQPREPQVYTLPPSRDELTKNQVSLWCLVKGFYPSDIAVEWESNGQPENNYKT TPPVLDSDGSFFLYSKLTVDKSRWQQGNVFSCSVMHEALHNHYTQKSLSLSPG K |
| Heavy chain constant region CH3 SEQ ID NO: 73 | GQPREPQVYTLPPSRDEL TKNQVSL TCL VKGF YPSDIA VEWESNGQPENNYDTT PPVLDSDGSFFL YSDLTVDKSRWQQGNVFSCSVMHEALHNHYTQKSLSLSPGK |
| Heavy chain constant region CH3 SEQ ID NO: 74 | GQPREPQVYTLPPSRDELTKNQVSLSCAVKGFYPSDIAVEWESNGQPENNYKTT PPVLDSDGSFFLVSKLTVDKSRWQQGNVFSCSVMHEALHNHYTQKSLSLSPGK |
| Heavy chain constant region CH3 SEQ ID NO: 75 | GQPREPQVCTLPPSRDELTKNQVSLSCAVKGFYPSDIAVEWESNGQPENNYKTT PPVLDSDGSFFLVSKLTVDKSRWQQGNVFSCSVMHEALHNRFTQKSLSLSPGK |
| Heavy chain constant region CH3 SEQ ID NO: 76 | GQPREPQVYTLPPSRKELTKNQVSLTCLVKGFYPSDIAVEWESNGQPENNYKTT PPVLKSDGSFFLYSKLTVDKSRWQQGNVFSCSVMHEALHNHYTQKSLSLSPGK |
| Anti-EpCAM VL (1) | ELVMTQSPSSLTVTAGEKVTMSCKSSQSLLNSGNQKNYLTWYQQKPGQPPKLLI YWASTRESGVPDRFTGSGSGTDFTLTISSVQAEDLAVYYCQNDYSYPLTFGAGT KLEIK (SEQ ID NO: 13) (the underlined parts indicate CDRs) |
| | CDRL1: KSSQSLLNSGNQKNYLT (SEQ ID NO: 32) |
| | CDRL2: WASTRES (SEQ ID NO: 33) |
| | CDRL3: QNDYSYPLT (SEQ ID NO: 34) |
| Anti-EpCAM VH (1) | EVQLLEQSGAELVRPGTSVKISCKASGYAFTNYWLGWVKQRPGHGLEWIGDIF PGSGNIHYNEKFKGKATLTADKSSSTAYMQLSSLTFEDSAVYFCARLRNWDEP MDYWGQGTTVTVSS (SEQ ID NO: 14) (the underlined parts indicate CDRs) |
| | CDRH1: GYAFTNYWLG (SEQ ID NO: 35) |
| | CDRH2: DIFPGSGNIHYNEKFKG (SEQ ID NO: 36) |
| | CDRH3: LRNWDEPMDY (SEQ ID NO: 37) |
| Anti-EpCAM VL (2) | EIVMTQSPATLSVSPGERATLSCRASQSVSSNLAWYQQKPGQAPRLIIYGASTTA SGIPARFSASGSGTDFTLTISSLQSEDFAVYYCQQYNNWPPAYTFGQGTKLEIK (SEQ ID NO: 15) (the underlined parts indicate CDRs) CDRL1: RASQSVSSNLA (SEQ ID NO: 38) CDRL2: GASTTAS (SEQ ID NO: 39) CDRL3: QQYNNWPPAYT (SEQ ID NO: 40) |
| Anti-EpCAM VH (2) | QVQLVQSGAEVKKPGSSVKVSCKASGGTFSSYAISWVRQAPGQGLEWMGGIIPI FGTANYAQKFQGRVTITADESTSTAYMELSSLRSEDTAVYYCARGLLWNYWG QGTLVTVSS (SEQ ID NO: 16) (the underlined parts indicate CDRs) |
| | CDRH1: SYAIS (SEQ ID NO: 41) |
| | CDRH2: GIIPIFGTANYAQKFQG (SEQ ID NO: 42) |
| | CDRH3: GLLWNY (SEQ ID NO: 43) |
| Anti-CD3 ScFv (1) | QVQLQQSGAELARPGASVKMSCKASGYTFTRYTMHWVKQRPGQGLEWIGYIN PSRGYTNYNQKFKDKATLTTDKSSSTAYMQLSSLTSEDSAVYYCARYYDDHYCL DYWGQGTTLTVSSGGGGSGGGGSGGGGSQIVLTQSPAIMSASPGEKVTMTCSAS SSVSYMNWYQQKSGTSPKRWIYDTSKLASGVPAHFRGSGSGTSYSLTISGMEAE DAATYYCQQWSSNPFTFGSGTKLEINR (SEQ ID NO: 17) (the underlined parts indicate CDRs) |
| Anti-CD3 ScFv (2) | DIKLQQSGAELARPGASVKMSCKTSGYTFTRYTMHWVKQRPGQGLEWIGYINP SRGYTNYNQKFKDKATLTTDKSSSTAYMQLSSLTSEDSAVYYCARYYDDHYCL DYWGQGTTLTVSSGGGGSGGGGSGGGGSDIQLTQSPAIMSASPGEKVTMTCRA SSSVSYMNWYQQKSGTSPKRWIYDTSKVASGVPYRFSGSGSGTSYSLTISSMEA EDAATYYCQQWSSNPLTFGAGTKLELK (SEQ ID NO: 18) (the underlined parts indicate CDRs) |
| | CDRH1: GYTFTRYTMH (SEQ ID NO: 44) |
| | CDRH2: YINPSRGYTNYNQKFKD (SEQ ID NO: 45) |
| | CDRH3: YYDDHYCLDY (SEQ ID NO: 46) |
| | CDRL1: RASSSVSYMN (SEQ ID NO: 47) |
| | CDRL2: DTSKVASG (SEQ ID NO: 48) |
| | CDRL3: QQWSSNPLT (SEQ ID NO: 49) |
| | VH: |
| | DIKLQQSGAELARPGASVKMSCKTSGYTFTRYTMHWVKQRPGQGLEWIGYINP SRGYTNYNQKFKDKATLTTDKSSSTAYMQLSSLTSEDSAVYYCARYYDDHYCLD YWGQGTTLTVSS (SEQ ID NO: 50) |
| | VL: |
| | DIQLTQSPAIMSASPGEKVTMTCRASSSVSYMNWYQQKSGTSPKRWIYDTSKV ASGVPYRFSGSGSGTSYSLTISSMEAEDAATYYCQQWSSNPLTFGAGTKLELK (SEQ ID NO: 51) |
| Anti-CD3 ScFv (3) | QVQLVESGGGVVQPGRSLRLSCAASGFTFSTYAMNWVRQAPGKGLEWVARIR SKYNNYATYYADSVKDRFTISRDDSKNTLYLQMNSLRAEDTAVYYCARHGNF GNSYVSWFAYWGQGTLVTVSSGGGGSGGGGSGGGGSQTVVTQEPSLTVSPGG TVTLTCRSSTGAVTTSNYANWVQQKPGQAPRGLIGGTNKRAPGVPARFSGSLL GGKAALTLSGVQPEDEAEYYCALWYSNLWVFGGGTKVEIK (SEQ ID NO: 19) (the underlined parts indicate CDRs) |
| | CDRH1: GFTFSTYAMN (SEQ ID NO: 52) |
| | CDRH2: RIRSKYNNYATYYADSVKD (SEQ ID NO: 53) |
| | CDRH3: HGNFGNSYVSWFAY (SEQ ID NO: 54) |
| | CDRL1: RSSTGAVTTSNYAN (SEQ ID NO: 55) |
| | CDRL2: GTNKRAP (SEQ ID NO: 56) |
| | CDRL3: ALWYSNLWV (SEQ ID NO: 57) |
| | VH:QVQLVESGGGVVQPGRSLRLSCAASGFTFSTYAMNWVRQAPGKGLEWVAR IRSKYNNYATYYADSVKDRFTISRDDSKNTLYLQMNSLRAEDTAVYYCARHGNF GNSYVSWFAYWGQGTLVTVSS (SEQ ID NO: 58) VL:QTVVTQEPSLTVSPGGTVTLTCRSSTGAVTTSNYANWVQQKPGQAPRGLIG GTNKRAPGVPARFSGSLLGGKAALTLSGVQPEDEAEYYCALWYSNLWVFGGG TKVEIK (SEQ ID NO: 59) |
| Anti-CD3 ScFv (4) | EVQLVESGGGLVQPGGSLRLSCKASGYTFTRYTMHWVRQAPGKGLEWIGYINP SRGYTNYNQKVKDRFTISRDNSKNTAFLQMDSLRPEDTGVYFCARYYDDHYCL DYWGQGTPVTVSSGGGGSGGGGSGGGGSDIQMTQSPSSLSASVGDRVTITCSAS SSVSYMNWYQQTPGKAPKRWIYDTSKLASGVPSRFSGSGSGTDYTFTISSLQPE DIATYYCQQWSSNPFTFGQGTKVEIKR (SEQ ID NO: 20) (the underlined parts indicate CDRs) |
| Anti-CD3 ScFv (5) | EVQLLESGGGLVQPGGSLRLSCAASGFTFSSFPMAWVRQAPGKGLEWVSTISTS GGRTYYRDSVKGRFTISRDNSKNTLYLQMNSLRAEDTAVYYCAKFRQYSGGFD YWGQGTLVTVSSGGGGSGGGGSGGGGSDIQLTQPNSVSTSLGSTVKLSCTLSSG NIENNYVHWYQLYEGRSPTTMIYDDDKRPDGVPDRFSGSIDRSSNSAFLTIHNV AIEDEAIYFCHSYVSSFNVFGGGTKLTVLRQP (SEQ ID NO: 21) (the underlined parts indicate CDRs) |
| Anti-CD3 ScFv (6) | QVQLVESGGGVVQPGRSLRLSCAASGFKFSGYGMHWVRQAPGKGLEWVAVI WYDGSKKYYVDSVKGRFTISRDNSKNTLYLQMNSLRAEDTAVYYCARQMGY WHFDLWGRGTLVTVSSGGGGSGGGGSGGGGSEIVLTQSPATLSLSPGERATLSC |
| | RASQSVSSYLAWYQQKPGQAPRLLIYDASNRATGIPARFSGSGSGTDFTLTISSL EPEDFAVYYCQQRSNWPPLTFGGGTKVEIKR (SEQ ID NO: 22) (the underlined parts indicate CDRs) |
| Anti-CD3 ScFv (7) | QVQLVQSGAEVKKPGASVKVSCKASGYTFISYTMHWVRQAPGQGLEWMGYIN PRSGYTHYNQKLKDKATLTADKSASTAYMELSSLRSEDTAVYYCARSAYYDY DGFAYWGQGTLVTVSSGGGGSGGGGSGGGGSDIQMTQSPSSLSASVGDRVTIT CSASSSVSYMNWYQQKPGKAPKRLIYDTSKLASGVPSRFSGSGSGTDFTLTISSL QPEDFATYYCQQWSSNPPTFGGGTKVEIKR (SEQ ID NO: 23) (the underlined parts indicate CDRs) |
| Anti-CD3 ScFv (8) | EVQLVESGGGLVQPGGSLRLSCAASGYSFTGYTMNWVRQAPGKGLEWVALIN PYKGVSTYNQKFKDRFTISVDKSKNTAYLQMNSLRAEDTAVYYCARSGYYGDS DWYFDVWGQGTLVTVSSGGGGSGGGGSGGGGSDIQMTQSPSSLSASVGDRVTI TCRASQDIRNYLNWYQQKPGKAPKLLIYYTSRLESGVPSRFSGSGSGTDYTLTIS SLQPEDFATYYCQQGNTLPWTFGQGTKVEIK (SEQ ID NO: 24) (the underlined parts indicate CDRs) |

| Control antibody name | Amino acid sequence |
|---|---|
| M700 monoclonal antibody | Light chain (SEQ ID NO: 25) |
| | ELVMTQSPSSLTVTAGEKVTMSCKSSQSLLNSGNQKNYLTWYQQKPGQPPKLLIYW ASTRESGVPDRFTGSGSGTDFTLTISSVQAEDLAVYYCQNDYSYPLTFGAGTKLEIKR TVAAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |
| | Heavy chain (SEQ ID NO: 26) |
| | EVQLLEQSGAELVRPGTSVKISCKASGYAFTNYWLGWVKQRPGHGLEWIGDIFPGSG NIHYNEKFKGKATLTADKSSSTAYMQLSSLTFEDSAVYFCARLRNWDEPMDYWGQ GTTVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK |
| M100 monoclonal antibody | Light chain (SEQ ID NO: 27) |
| | DIQLTQSPAIMSASPGEKVTMTCRASSSVSYMNWYQQKSGTSPKRWIYDTSKVASGV PYRFSGSGSGTSYSLTISSMEAEDAATYYCQQWSSNPLTFGAGTKLELKRTVAAPSVF IFPPSDEQLKSGTASVVCLLNNFYPREAKVQWKVDNALQSGNSQESVTEQDSKDSTY SLSSTLTLSKADYEKHKVYACEVTHQGLSSPVTKSFNRGEC |
| | Heavy chain (SEQ ID NO: 28) |
| | DIKLQQSGAELARPGASVKMSCKTSGYTFTRYTMHWVKQRPGQGLEWIGYINPSRG YTNYNQKFKDKATLTTDKSSSTAYMQLSSLTSEDSAVYYCARYYDDHYCLDYWGQ GTTLTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK |
| Mco101 | Light chain (SEQ ID NO: 29) |
| | DVVMTQTPLSLPVSLGDQASISCRSSQSLVHSNGNTYLRWYLQKPGQSPKVLIYKVS NRFSGVPDRFSGSGSGTDFTLKISRVEAEDLGVYFCSQSTHVPWTFGGGTKLEIKRTV AAPSVFIFPPSDEQLKSGTASVVCLLNNFYPREAKVQWKVDNALQSGNSQESVTEQD SKDSTYSLSSTLTLSKADYEKHKVYACEVTHQGLSSPVTKSFNRGEC |
| | Heavy chain (SEQ ID NO: 30) |
| | EVKLDETGGGLVQPGRPMKLSCVASGFTFSDYWMNWVRQSPEKGLEWVAQIRNKP YNYETYYSDSVKGRFTISRDDSKSSVYLQMNNLRVEDMGIYYCTGSYYGMDYWGQ GTSVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKA |
| | KGQPREPQVYTLPPSRDELTKNQVSLWCLVKGFYPSDIAVEWESNGQPENNYDTTPP VLDSDGSFFLYSDLTVDKSRWQQGNVFSCSVMHEALHNHYTQKSLSLSPGK |
| | Single chain (SEQ ID NO: 31) |
| | DIKLQQSGAELARPGASVKMSCKTSGYTFTRYTMHWVKQRPGQGLEWIGYINPSRG YTNYNQKFKDKATLTTDKSSSTAYMQLSSLTSEDSAVYYCARYYDDHYCLDYWGQ GTTLTVSSGGGGSGGGGSGGGGSDIQLTQSPAIMSASPGEKVTMTCRASSSVSYMNW YQQKSGTSPKRWIYDTSKVASGVPYRFSGSGSGTSYSLTISSMEAEDAATYYCQQWS SNPLTFGAGTKLELKGAAAEPKSCDKTHTCPPCPAPELLGGPSVFLFPPKPKDTLMISR TPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSRDELTKNQVSLTC RVKGFYPSDIAVEWESNGQPENNYKTTPPVLKSDGSFFLASKLTVDKSRWQQGNVFS CSVMHEALHNHYTQKSLSLSPGK |

## Claims

1. A bispecific antibody, comprising an antigen-binding domain specifically binding to EpCAM and an antigen-binding domain specifically binding to CD3,
wherein the antigen-binding domain specifically binding to EpCAM is selected from the group consisting of:
1) an antigen-binding domain specifically binding to EpCAM, which comprises the following CDRs or variants thereof:
(i) CDRH1, CDRH2 and CDRH3 comprised in a heavy chain variable region set forth in SEQ ID NO: 14, and
(ii) CDRL1, CDRL2 and CDRL3 comprised in a light chain variable region set forth in SEQ ID NO: 13, wherein preferably, according to the Kabat sequence numbering system, the sequence of CDRL1 is set forth in SEQ ID NO: 32, the sequence of CDRL2 is set forth in SEQ ID NO: 33, the sequence of CDRL3 is set forth in SEQ ID NO: 34, the sequence of CDRH1 is set forth in SEQ ID NO: 35, the sequence of CDRH2 is set forth in SEQ ID NO: 36, and the sequence of CDRH3 is set forth in SEQ ID NO: 37; or
2) an antigen-binding domain specifically binding to EpCAM, which comprises the following CDRs or variants thereof:
(i) CDRH1, CDRH2 and CDRH3 comprised in a heavy chain variable region set forth in SEQ ID NO: 16, and
(ii) CDRL1, CDRL2 and CDRL3 comprised in a light chain variable region set forth in SEQ ID NO: 15,
wherein preferably, according to the Kabat sequence numbering system and CDR definition system, the sequence of CDRL1 is set forth in SEQ ID NO: 38, the sequence of CDRL2 is set forth in SEQ ID NO: 39, the sequence of CDRL3 is set forth in SEQ ID NO: 40, the sequence of CDRH1 is set forth in SEQ ID NO: 41, the sequence of CDRH2 is set forth in SEQ ID NO: 42, and the sequence of CDRH3 is set forth in SEQ ID NO: 43;
the antigen-binding domain specifically binding to CD3 is selected from the group consisting of:
1) an antigen-binding domain specifically binding to CD3, which comprises the following CDRs or variants thereof:
CDRH1, CDRH2 and CDRH3 comprised in a heavy chain variable region set forth in SEQ ID NO: 50, and CDRL1, CDRL2 and CDRL3 comprised in a light chain variable region set forth in SEQ ID NO: 51,
wherein preferably, according to the Kabat sequence numbering system, the sequence of CDRH1 is set forth in SEQ ID NO: 44, the sequence of CDRH2 is set forth in SEQ ID NO: 45, the sequence of CDRH3 is set forth in SEQ ID NO: 46, the sequence of CDRL1 is set forth in SEQ ID NO: 47, the sequence of CDRL2 is set forth in SEQ ID NO: 48, and the sequence of CDRL3 is set forth in SEQ ID NO: 49; or
2) an antigen-binding domain specifically binding to CD3, which comprises the following CDRs or variants thereof:
CDRH1, CDRH2 and CDRH3 comprised in a heavy chain variable region set forth in SEQ ID NO: 58, and CDRL1, CDRL2 and CDRL3 comprised in a light chain variable region set forth in SEQ ID NO: 59,
wherein preferably, according to the Kabat sequence numbering system, the sequence of CDRH1 is set forth in SEQ ID NO: 52, the sequence of CDRH2 is set forth in SEQ ID NO: 53, the sequence of CDRH3 is set forth in SEQ ID NO: 54, the sequence of CDRL1 is set forth in SEQ ID NO: 55, the sequence of CDRL2 is set forth in SEQ ID NO: 56, and the sequence of CDRL3 is set forth in SEQ ID NO: 57; the variants of the CDRs differ from the corresponding CDRs by 3, 2 or 1 amino acid or have at least 80%, 85%, 90%, 91%, 92%, 93%, 94%, 95%, 96%, 97%, 98% or 99% identity to the corresponding CDRs;
preferably, the antigen-binding domain specifically binding to EpCAM is in the form of a Fab fragment and the antigen-binding domain specifically binding to CD3 is in the form of an ScFv.

2. The bispecific antibody according to claim 1, wherein the antigen-binding domain specifically binding to EpCAM comprises the following heavy chain variable region and light chain variable region (or variants thereof):
(i) a heavy chain variable region set forth in SEQ ID NO: 14 and a light chain variable region set forth in SEQ ID NO: 13; or
(ii) a heavy chain variable region set forth in SEQ ID NO: 16 and a light chain variable region set forth in SEQ ID NO: 15; and
the antigen-binding domain specifically binding to CD3 comprises the following heavy chain variable region and light chain variable region (or variants thereof):
(1) a heavy chain variable region set forth in SEQ ID NO: 50 and a light chain variable region set forth in SEQ ID NO: 51, or
(2) a heavy chain variable region set forth in SEQ ID NO: 58 and a light chain variable region set forth in SEQ ID NO: 59;
preferably,
the antigen-binding domain specifically binding to EpCAM comprises the following heavy chain variable region and light chain variable region (or variants thereof):
(i) a heavy chain variable region set forth in SEQ ID NO: 14 and a light chain variable region set forth in SEQ ID NO: 13; and
the antigen-binding domain specifically binding to CD3 is selected from the group consisting of:
(1) an ScFv set forth in SEQ ID NO: 18 or a variant thereof,
(2) an ScFv set forth in SEQ ID NO: 19 or a variant thereof,
wherein the variants differ from the corresponding variable regions or ScFvs by 3, 2 or 1 amino acid or have at least 80%, 85%, 90%, 91%, 92%, 93%, 94%, 95%, 96%, 97%, 98% or 99% identity to the corresponding variable regions or ScFvs.

3. The bispecific antibody according to claim 1 or 2, wherein the bispecific antibody comprises:
(1) a light chain-heavy chain pair specifically binding to EpCAM, the light chain-heavy chain pair comprising or consisting of a light chain and a heavy chain, wherein the light chain comprises a light chain variable region and a light chain constant region (a preferred sequence is set forth in any one of SEQ ID NOs: 1 and 60-65), and the heavy chain comprises a heavy chain variable region, CH1 (a preferred sequence is set forth in SEQ ID NO: 2) and a first Fc fragment, wherein preferably, the first Fc fragment comprises a hinge region (a preferred sequence is set forth in SEQ ID NO: 3), CH2 (a preferred sequence is set forth in any one of SEQ ID NOs: 6, 7 and 66-71) and CH3a; and
(2) a fusion peptide specifically binding to CD3, the fusion peptide comprising or consisting of an ScFv specifically binding to CD3 and a second Fc fragment, wherein preferably, the ScFv comprises, in sequence from the N-terminus to the C-terminus, a heavy chain variable region, a linker peptide (a preferred sequence is set forth in SEQ ID NO: 4) and a light chain variable region, and the second Fc fragment comprises, in sequence from the N-terminus to the C-terminus, a hinge region (a preferred sequence is set forth in SEQ ID NO: 3), CH2 (a preferred sequence is set forth in any one of SEQ ID NOs: 6, 7 and 66-71) and CH3b, wherein preferably, the C-terminus of the light chain variable region is linked to the hinge region of the second Fc fragment via a linker peptide (a preferred sequence is set forth in SEQ ID NO: 5);
preferably, the first Fc fragment and the second Fc fragment are human or humanized Fc fragments, such as human IgG Fc fragments, e.g., IgG1, IgG2, IgG3, IgG4 or IgG5 Fc fragments;
preferably, compared to a wild-type antibody, the first Fc fragment and/or the second Fc fragment comprise one or more substitutions, that form a knob-into-hole structure pair between the heavy chain and the fusion peptide, e.g., T366 on one CH3 domain is substituted with a relatively larger amino acid residue, such as tyrosine (Y) or tryptophan (W), while Y407 on the other CH3 domain is substituted with a relatively smaller amino acid residue, such as threonine (T), alanine (A) or valine (V); for example, the first Fc fragment and/or the second Fc fragment comprise one or more substitutions in Table 6; preferably, the first Fc fragment and/or the second Fc fragment comprise one or more substitutions, wherein 1) the substitution forms a salt bridge pair between the heavy chain and the fusion peptide, e.g., one CH3 domain comprises one or more substitutions with an amino acid residue that is positively charged under physiological conditions, while the other CH3 domain comprises one or more substitutions with one or more amino acid residues that are negatively charged under physiological conditions, wherein the positively charged amino acid residue is, for example, arginine (R), histidine (H) or lysine (K), the negatively charged amino acid residue may be, for example, aspartic acid (D) or glutamic acid (E), and the substituted amino acid residues comprise, for example, one or more of D356, L368, K392, D399 and K409, for example, one or more substitutions in Table 7; 2) the substitution forms a disulfide bond between the heavy chain and the fusion peptide, for example the substitutions in Table 8; and/or 3) the substitution results in a significant reduction in binding ability between the Fc and protein A, for example, H435 and Y436 on one CH3 domain are substituted with arginine and phenylalanine, respectively, as shown in Table 9; wherein preferably:
a) the CH3b of the fusion peptide and the CH3a of the heavy chain have a substitution pair that forms a knob-into-hole structure;
b) the CH3b of the fusion peptide and the CH3a of the heavy chain have a substitution pair that forms an ionic bond;
c) the CH3b of the fusion peptide and the CH3a of the heavy chain have a substitution pair that forms a disulfide bond; and/or
d) the CH3b of the fusion peptide and the CH3a of the heavy chain have a substitution that results in reduced binding ability to the protein A;
preferably, the CH1 comprises a sequence of SEQ ID NO: 2; and/or CL comprises a sequence selected from any one of SEQ ID NOs: 1 and 60-65;
preferably, the first Fc fragment and/or the second Fc fragment comprise CH2 of a sequence selected from any one of SEQ ID NOs: 6, 7 and 66-71, and/or CH3 of a sequence selected from any one of SEQ ID NOs: 8, 9, 11, 12 and 72-76;
preferably, the sequences of CH3a and CH3b are selected from the group consisting of:
(1) sequences in which one is set forth in SEQ ID NO: 8 and the other is set forth in SEQ ID NO: 11;
(2) sequences in which one is set forth in SEQ ID NO: 9 and the other is set forth in SEQ ID NO: 12;
(3) sequences in which one is set forth in SEQ ID NO: 72 and the other is set forth in SEQ ID NO: 74;
(4) sequences in which one is set forth in SEQ ID NO: 9 and the other is set forth in SEQ ID NO: 75;
(5) sequences in which one is set forth in SEQ ID NO: 73 and the other is set forth in SEQ ID NO: 76;
preferably, the bispecific antibody is selected from the group consisting of:
(1) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 11, the heavy chain comprises or consists of SEQ ID NO: 14, SEQ ID NO: 2, SEQ ID NO: 3, SEQ ID NO: 6 and SEQ ID NO: 8, and the light chain comprises or consists of SEQ ID NO: 13 and SEQ ID NO: 1;
(2) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 11, the heavy chain comprises or consists of SEQ ID NO: 14, SEQ ID NO: 2, SEQ ID NO: 3, SEQ ID NO: 6 and SEQ ID NO: 8, and the light chain comprises or consists of SEQ ID NO: 13 and SEQ ID NO: 1;
(3) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 11, the heavy chain comprises or consists of SEQ ID NO: 16, SEQ ID NO: 2, SEQ ID NO: 3, SEQ ID NO: 6 and SEQ ID NO: 8, and the light chain comprises or consists of SEQ ID NO: 15 and SEQ ID NO: 1;
(4) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 11, the heavy chain comprises or consists of SEQ ID NO: 16, SEQ ID NO: 2, SEQ ID NO: 3, SEQ ID NO: 6 and SEQ ID NO: 8, and the light chain comprises or consists of SEQ ID NO: 15 and SEQ ID NO: 1;
(5) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 7 and SEQ ID NO: 12, the heavy chain comprises or consists of SEQ ID NO: 14, SEQ ID NO: 2, SEQ ID NO: 3, SEQ ID NO: 7 and SEQ ID NO: 9, and the light chain comprises or consists of SEQ ID NO: 13 and SEQ ID NO: 1;
(6) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 7 and SEQ ID NO: 12, the heavy chain comprises or consists of SEQ ID NO: 14, SEQ ID NO: 2, SEQ ID NO: 3, SEQ ID NO: 7 and SEQ ID NO: 9, and the light chain comprises or consists of SEQ ID NO: 13 and SEQ ID NO: 1;
(7) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 8, the heavy chain comprises or consists of SEQ ID NO: 14, SEQ ID NO: 2, SEQ ID NO: 3, SEQ ID NO: 6 and SEQ ID NO: 11, and the light chain comprises or consists of SEQ ID NO: 13 and SEQ ID NO: 1;
(8) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 8, the heavy chain comprises or consists of SEQ ID NO: 14, SEQ ID NO: 2, SEQ ID NO: 3, SEQ ID NO: 6 and SEQ ID NO: 11, and the light chain comprises or consists of SEQ ID NO: 13 and SEQ ID NO: 1;
(9) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 6 and SEQ ID NO: 8, the heavy chain comprises or consists of SEQ ID NO: 16, SEQ ID NO: 2, SEQ ID NO: 3, SEQ ID NO: 6 and SEQ ID NO: 11, and the light chain comprises or consists of SEQ ID NO: 15 and SEQ ID NO: 1;
(10) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 6 and SEQ ID NO: 8, the heavy chain comprises or consists of SEQ ID NO: 16, SEQ ID NO: 2, SEQ ID NO: 3, SEQ ID NO: 6 and SEQ ID NO: 11, and the light chain comprises or consists of SEQ ID NO: 15 and SEQ ID NO: 1;
(11) a bispecific antibody comprising or consisting of a fusion peptide, a heavy chain, and a light chain, wherein the fusion peptide comprises or consists of SEQ ID NO: 18, SEQ ID NO: 5, SEQ ID NO: 3, SEQ ID NO: 7 and SEQ ID NO: 9, the heavy chain comprises or consists of SEQ ID NO: 14, SEQ ID NO: 2, SEQ ID NO: 3, SEQ ID NO: 7 and SEQ ID NO: 12, and the light chain comprises or consists of SEQ ID NO: 13 and SEQ ID NO: 1; and
(12) a bispecific antibody comprising or consisting of a fusion peptide, a heavy chain, and a light chain, wherein the fusion peptide comprises or consists of SEQ ID NO: 19, SEQ ID NO: 5, SEQ ID NO: 3, SEQ ID NO: 7 and SEQ ID NO: 9, the heavy chain comprises or consists of SEQ ID NO: 14, SEQ ID NO: 2, SEQ ID NO: 3, SEQ ID NO: 7 and SEQ ID NO: 12, and the light chain comprises or consists of SEQ ID NO: 13 and SEQ ID NO: 1.

4. A nucleic acid composition, comprising a nucleic acid sequence encoding the bispecific antibody according to any one of claims 1-3, wherein preferably,
the nucleic acid composition comprises:
a) a first expression vector comprising a first nucleic acid encoding the antigen-binding domain or light chain-heavy chain pair specifically binding to EpCAM as defined in any one of claims 1-3;
b) a second expression vector comprising a second nucleic acid encoding the antigen-binding domain or fusion peptide specifically binding to CD3 as defined in any one of claims 1-3.

5. An expression vector, comprising the nucleic acid composition according to claim 4.

6. A host cell, comprising the expression vector according to claim 5.

7. A pharmaceutical composition, comprising the bispecific antibody according to any one of claims 1-3, and a pharmaceutically acceptable carrier, and optionally, a drug (e.g., a small molecule drug or a large molecule drug) for treating cancer (an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, oral cancer, or the like) and/or malignant ascites, malignant effusion, malignant pleural effusion, or the like, wherein preferably, the dosage form of the pharmaceutical composition comprises a dosage form for gastrointestinal administration or a dosage form for parenteral administration; and more preferably, the dosage form of the pharmaceutical composition is an injectable form, including intravenous injection, intravenous drip, subcutaneous injection, topical injection, intramuscular injection, intratumoral injection, intraperitoneal injection, intracranial injection, or intracavitary injection.

8. A conjugate or fusion protein, comprising the bispecific antibody according to any one of claims 1-3, preferably comprising a substance A conjugated or fused to the bispecific antibody, wherein the substance A is selected from a therapeutic agent, a prodrug, a protein (e.g., an enzyme), a virus, a lipid, a biological response modifier (e.g., an immunomodulator), PEG, a hormone, an oligonucleotide, a diagnostic agent, a cytotoxic agent that can be a drug or a toxin, an ultrasound enhancing agent, a non-radioactive label, a detectable label such as a chemiluminescent labeling compound (e.g., luminol, isoluminol, theromatic acridinium ester, imidazole, acridinium salt, and oxalate), or a fluorescent luminescent metal (e.g., 152Eu, or a lanthanide label).

9. A kit, comprising the bispecific antibody according to any one of claims 1-3 and optionally, a drug (e.g., a small molecule drug or a large molecule drug) for treating cancer (an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, oral cancer, or the like) and/or malignant ascites, malignant effusion, malignant pleural effusion, or the like.

10. Use of the bispecific antibody according to any one of claims 1-3 in the treatment of cancer or in the preparation of a medicament or kit for treating cancer and/or malignant ascites, malignant effusion, malignant pleural effusion, or the like, wherein the cancer is, for example, an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, or oral cancer.

11. A method for treating cancer and/or malignant ascites, malignant effusion, or malignant pleural effusion, comprising administering to a subject a therapeutically effective amount of the bispecific antibody according to any one of claims 1-3, wherein the cancer is an EpCAM-positive tumor, such as colorectal cancer, gastric cancer, breast cancer, ovarian cancer, lung cancer (e.g., non-small cell lung cancer), prostate cancer, pancreatic cancer, liver cancer, retinoblastoma, esophageal cancer, renal cancer, clear cell renal cell carcinoma, skin squamous cell carcinoma, skin basal cell carcinoma, sarcoma, esthesioneuroblastoma, craniopharyngioma, thyroid cancer, cholangiocarcinoma, bladder cancer, head and neck tumor, cervical cancer, or oral cancer.
